# EUROPÄISCHE PATENTANMELDUNG

(11) **EP 4 736 891 A2**
(43) Veröffentlichungstag der Anmeldung: **06.05.2026**
(21) Anmeldenummer: 25202625.7
(22) Anmeldetag: 17.09.2025
(51) Int. Cl.: A61L 2/07

(54) **VERFAHREN ZUM STERILISIEREN UND STERILEN LAGERN MEDIZINISCHER INSTRUMENTE**

(30) Priorität: 17.09.2024 CH 10362024; 19.09.2024 CH 10462024; 04.10.2024 CH 10962024
(71) Anmelder: Dr. med. dent. Fässler, Daniel, 5400 Badench (CH)
(72) Erfinder: FÄSSLER, Daniel, Dr. med. dent., 5400 Baden (CH); HAGMANN, Peter, 4574 Nennigkofen (CH)
(74) Vertreter: Körner, Thomas Ottmar

(57) **Zusammenfassung**

Die Erfindung betrifft ein Verfahren zum Sterilisieren von medizinischen Instrumenten, umfassend die Schritte
e. Einbringen von Sterilisationsgut umfassend zu sterilisierende medizinische Instrumente in einen ersten Innenraum (11) eines Sterilisators (1), insbesondere eines Autoklaven;
f. Sterilisieren des Sterilisationsguts im Sterilisator, insbesondere mittels eines Sterilisierungsprozesses des Sterilisators, um Sterilgut umfassend die medizinischen Instrumente zu erhalten;
g. Entnehmen des Sterilguts durch eine Entladeöffnung (12) des Sterilisators;
h. Einbringen des Sterilguts in einen zweiten Innenraum (21) einer Sicherheitswerkbank (2), insbesondere einer Laminar Flow Box durch eine Befüllungsöffnung (23) der Sicherheitswerkbank;
i. Lagern des Sterilguts im zweiten Innenraum (21) der Sicherheitswerkbank;
j. Entnehmen des Sterilguts durch eine Entnahmeöffnung der Sicherheitswerkbank; wobei
k. die Entladeöffnung mit der Befüllungsöffnung verbunden ist oder wird, insbesondere derart, dass die Entladeöffnung in die Befüllungsöffnung mündet und/oder geführt ist, insbesondere in die Befüllungsöffnung hineinragt; und
I. das Sterilgut dem zweiten Innenraum erst unmittelbar vor einem medizinischen, insbesondere zahnmedizinischen, Eingriff entnommen wird, bei welchem die medizinischen Instrumente eingesetzt werden, wobei
m. das Sterilisationsgut auf oder in einem oder mehreren Trägern, insbesondere in Form von (wieder-)verschliessbaren Boxen, in den ersten Innenraum eingebracht wird, wobei insbesondere eine Vielzahl von unterschiedlichen Trägern und/oder Trägern unterschiedlicher Art vorhanden sind oder verwendet werden, welche sich insbesondere dadurch unterscheiden, dass sie dazu ausgelegt sind, ein unterschiedliches Sortiment an medizinischen Instrumenten aufzunehmen, wobei insbesondere eine erste Art von Träger dazu ausgelegt ist, ein erstes Sortiment aufzunehmen, und eine zweite Art von Träger dazu ausgelegt ist, ein zweites Sortiment aufzunehmen, welches sich von dem ersten Sortiment unterscheidet.

## Beschreibung

Die Erfindung bezieht sich auf eine für eine Vorbereitung und Handhabung medizinsicher, insbesondere zahnmedizinischer, Instrumente notwendige Logistik. Sie betrifft ein System und Verfahren zum Sterilisieren und sterilen Aufbewahren und/oder Lagern medizinischer Instrumente sowie ein Verfahren zum Bereitstellen eines entsprechenden Systems gemäss den unabhängigen Patentansprüchen.

### Hintergrund der Erfindung

Durch die neuen Hygienevorschriften für Zahnarztpraxen oder Ähnliches (z.B. Podologie) entsteht viel mehr Arbeit und Abfall.

Insbesondere in der zahnärztlichen Praxis können Instrumente und Werkzeuge hinsichtlich hygienischer Anforderungen in drei Klassen eingeteilt werden: kritische Instrumente bzw. Werkzeuge, halbkritische Instrumente bzw. Werkzeuge, und nicht-kritische Instrumente bzw. Werkzeuge.

Insbesondere jedes kritische Werkzeug, Instrument etc. muss vor dem Einbringen in einen Sterilisator, insbesondere einen Autoklaven, in welchem eine Sterilisation von Instrumenten und Werkzeugen in der (zahn-)ärtzlichen Praxis in der Regel erfolgt, in einen Beutel, insbesondere einen sogenannten Sterilisationsbeutel, eingeschweisst und vor dessen Gebrauch, insbesondere erst unmittelbar vor Gebrauch, wieder ausgepackt werden.

Alles, was eingepackt werden muss, hat auch mehr Volumen und erschwert eine Übersicht in der Lagerhaltung in der Praxis. Die Sicht zu den Werkzeugen etc. ist viel eingeschränkter. Zudem entsteht eine Abhängigkeit von einer Verfügbarkeit und/oder zu den Lieferanten der Beutel.

Durch das erhöhte Volumen wird eine Kapazität eines Sterilisationsprozesses bzw. eines vorhandenen Sterilisators reduziert, insbesondere können pro Sterilisationsprozess und somit pro Zeiteinheit eine wesentlich geringere Anzahl Werkzeuge, Instrumente etc. sterilisiert werden, als dies bei einer Sterilisation in nicht eingeschweisstem Zustand der Fall wäre. Dies ist auch darauf zurückzuführen, dass Sterilisationsbeutel mit eingeschweissten Werkzeugen, Instrumenten etc. mit einem grösseren Abstand zueinander im Autoklaven positioniert werden müssen als dies bei nicht eingeschweissten Werkzeugen, Instrumenten etc. der Fall wäre, da ansonsten ein unbefriedigendes Sterilisationsergebnis resultieren kann.

Auch in eingeschweisstem Zustand, d.h. im Sterilisationsbeutel, können kritische Werkzeuge, Instrumente etc. jedoch nicht zeitlich unbegrenzt aufbewahrt werden. Dies liegt Daran, dass die Sterilisationsbeutel im Laufe der Zeit undicht werden, was insbesondere auf kleinere, teilweise mit blossem Auge nicht sichtbare Beschädigungen wie Löcher oder Schnitte zurückzuführen sein kann. Diese können mehr oder weniger zwangsläufig entstehen, wenn die Sterilisationsbeutel mit den grösstenteils spitzen und oder scharfen Werkzeuge, Instrumente etc. behändigt oder anderweitig bewegt werden, insbesondere beim Herausnehmen einzelner Sterilisationsbeutel aus einer Ansammlung z.B. in einem Sortimentskasten oder einer Sortimentsbox, bei der Lagerung in Schubladen bei deren Öffnen und Schliessen, usw. Teilweise werden die Sterilisationsbeutel, auch mit Blick auf die vorstehend geschilderten Probleme, mit einem Verfalldatum versehen, nach welchem die enthaltenen Werkzeuge, Instrumente etc. nicht mehr verwendet werden dürfen.

Wegen einer eingeschränkten Sichtbarkeit der Werkzeuge, Instrumente etc. im Sterilisationsbeutel kommt es auch immer wieder vor, dass versehentlich ein falsches, insbesondere für einen beabsichtigten Verwendungszweck ungeeignetes oder zumindest suboptimales Werkzeug, Instrument etc. ausgepackt wird.

In den vorgenannten Fällen müssen die Werkzeuge, Instrumente etc. erneut eingeschweisst und einem Sterilisationsprozess unterzogen werden, bevor sie erneut verwendet werden können. Dies führt zu einem an sich unnötigen Bedarf an Ressourcen, einerseits in Form von Rohstoffen wie für die Herstellung der Sterilisationsbeutel benötigtem Kunststoff und somit letztendlich Erdöl, andererseits in Form von Energie sowohl für die Herstellung der Sterilisationsbeutel als auch für den Sterilisationsprozess selbst. Ferner entsteht unnötiger Abfall in Form zusätzlich benötigter Sterilisationsbeutel. Schliesslich wird Arbeitskraft gebunden, um beschädigte und/oder abgelaufene Sterilisationsbeutel auszusortieren, und die nicht zum Einsatz gekommen Werkzeuge, Instrumente etc. erneut einzuschweissen und zu sterilisieren.

Der Erfindung liegt daher die Aufgabe zugrunde, ein System und ein Verfahren zum Sterilisieren und sterilen Lagern medizinischer Instrumente und Werkzeuge anzugeben, welche die vorstehend geschilderten Nachteile vermeidet oder vermindert, sowie ein Verfahren zum Bereitstellen eines entsprechenden Systems.

### Kurze Beschreibung der Erfindung

Diese Aufgabe kann erfindungsgemäss durch ein System und Verfahren zum Sterilisieren und sterilen Lagern medizinischer Instrumente und/oder Werkzeuge sowie ein Verfahren zum Bereitstellen eines entsprechenden Systems gemäss den unabhängigen Patentansprüchen gelöst werden.

In den nachfolgenden Ausführungen, insbesondere den Patentansprüchen, wird der Begriff Instrumente sowohl für Instrumente als auch für Werkzeuge verwendet.

System und Verfahren beruhen auf der Idee, Instrumente, welche vor einem Einsatz im Rahmen einer Behandlung eines Patienten sterilisiert werden müssen, und insbesondere bis unmittelbar vor diesem Einsatz in sterilem Zustand gehalten werden müssen, in unverpacktem, insbesondere nicht eingeschweissten Zustand in einem Sterilisator, insbesondere einem ersten Innenraum eines Sterilisators, zu sterilisieren, und nach erfolgter Sterilisation direkt und unmittelbar in eine Sicherheitswerkbank, insbesondere einen zweiten Innenraum einer Sicherheitswerkbank, zu verbringen, und diese dort bis unmittelbar vor ihrem Einsatz zu lagern. Um dies zu ermöglichen, kann an der Sicherheitswerkbank, bei der es sich insbesondere um eine Laminar Flow Box handeln kann, zusätzlich zu einer herkömmlichen Arbeitsöffnung eine separate Befüllungsöffnung vorgesehen sein. Eine Entladeöffnung des Sterilisators, welche insbesondere bei einem einfachen, herkömmlichen und/oder handelsüblichen Sterilisator auch der Beladung dienen kann. Die Entladeöffnung kann dabei derart mit der Befüllungsöffnung verbunden sein oder werden, dass die Entladeöffnung in die Befüllungsöffnung mündet und/oder geführt ist, insbesondere in die Befüllungsöffnung hineinragt. Dies erlaubt es, sterilisierte Instrumente dem Sterilisator zu entnehmen und in den zweiten Innenraum der Sicherheitswerkbank zu verbringen, ohne dass diese eine nicht sterile Umgebung durchqueren müssen, in welcher Sterilisator uns Sicherheitswerkbank aufgestellt sind.

Zu sterilisierende Instrumente, insbesondere Instrumente, die im Rahmen einer Behandlung eines Patienten eingesetzt wurden, können nachfolgend als Sterilisationsgut bezeichnet werden. Nach erfolgter Sterilisation sind diese erneut einsatzbereit, und werden als Sterilgut bezeichnet.

Ein erfindungsgemässes System zum Sterilisieren und zum sterilen Lagern und/oder Aufbewahren medizinischer Instrumente, welchem zu sterilisierende medizinische, insbesondere zahnmedizinische, Instrumente als Sterilisationsgut zugeführt und sterilisierte medizinische Instrumente als Sterilgut entnommen werden können, kann insbesondere umfassen:
a. einen Sterilisator (1), insbesondere einen Autoklaven, umfassend:
   i. einen ersten Innnenraum (11) zur Aufnahme von Sterilisationsgut,
   ii. eine Entladeöffnung (12) zum Entnehmen von Sterilgut aus dem ersten Innenraum,
   iii. eine Entladeklappe (121), mittels welcher insbesondere die Entladeöffnung fluid- und druckdicht verschlossen werden kann;
b. eine Sicherheitswerkbank (2), insbesondere eine Laminar Flow Box, umfassend:
   i. einen zweiten Innenraum (21), insbesondere zur Aufbewahrung von Sterilgut,
   ii. eine Befüllungsöffnung (23) zur Befüllung des zweiten Innenraums mit Sterilgut,
   iii. eine Entnahmeöffnung zur Entnahme von Sterilgut aus dem zweiten Innenraum; wobei
c. die Entladeöffnung mit der Befüllungsöffnung verbunden ist, insbesondere in die Befüllungsöffnung mündet und/oder geführt ist, insbesondere in die Befüllungsöffnung hineinragt.

Der Sterilisator kann dazu ausgelegt sein, in bzw. während eines Sterilisationsprozesses oder -programms in seinem ersten Innenraum Bedingungen einzustellen, die zumindest mit an Sicherheit grenzender Wahrscheinlichkeit Organismen, Keime, Bakterien, Viren, Sporen, Prionen und/oder weitere Krankheitserreger, welche sich ersten Innenraum und/oder auf in diesem befindlichen Instrumenten abtöten und/oder zerstören, insbesondere während einer ersten Zeitspanne, welche kürzer ist als eine zweite Zeitspanne von einem Beginn bis zu einem Ende des Sterilisationsprozesses oder -programms. Die genannten Bedingungen können dabei insbesondere eine Temperatur oberhalb von 100°C umfassen, insbesondere von oder oberhalb von 121°C.

Beim Sterilisator kann es sich dabei insbesondere um eine Vorrichtung handeln, mittels welcher eine Sterilisation, insbesondere ein Sterilisationsprozess, wie in den Wikipedia Artikeln https://en.wikipedia.org/w/index.php?title=Sterilization_(microbiology)&oldid=1158241630
und
https://de.wikipedia.org/w/index.php?title=Sterilisation&oldid=234122836
   beschrieben, durchgeführt werden kann; insbesondere um einen Autoklaven wie in den Wikipedia Artikeln
https://de.wikipedia.org/w/index.php?title=Autoklav&oldid=229184556
   und
https://en.wikipedia.org/w/index.php?title=Autoclave&oldid=1145034987
   beschrieben. Insbesondere kann es sich beim Autoklaven um einen herkömmlichen und/oder handelsüblichen Autoklaven handeln, wie er in kleineren ärztlichen oder zahnärztlichen Praxen, insbesondere mit maximal 5 Ärzten bzw. Zahnärzten zum Einsatz kommt. Ein derartiger Autoklav weist in der Regel eine einzige Öffnung auf, durch welche er mit Sterilisationsgut beladen werden kann, und durch welche nach erfolgter Sterilisation das Sterilgut entnommen werden kann. Die einzige Öffnung kann somit als Entladeöffnung bezeichnet werden.

Der Sterilisator kann eine Auswahl von Sollwerten für bestimmte Prozessparameter erlauben, insbesondere betreffend eine minimale, erforderliche Temperatur und/oder Dauer des Sterilisationsprozesses. Die Auswahl kann durch eine Einstellung eines bestimmten Sterilisationsprogramms erfolgen, welches aus einer Vielzahl möglicher Programme ausgewählt werden kann, beispielsweise über einen Knopf, einen Schalter oder ein am Sterilisator vorgesehenen berührungsempfindlichen Bildschirm zur Anzeige einer graphischen Benutzer- und/oder Bedienoberfläche.

Der Sterilisator kann ferner einen oder mehrere Sensoren umfassen, welche zumindest einen Teil der Prozessparameter, insbesondere deren aktuelle Istwerte, messen und/oder erfassen können, wie insbesondere Temperatur, Druck, (Luft-)Feuchtigkeit, insbesondere in Abhängigkeit von der Zeit. Ferner können ein oder mehrere AD-Wandler vorhanden sein, welche die gemessenen oder erfassten Werte in digitaler Form ausgeben können.

Bei der Sicherheitswerkbank kann es sich dabei insbesondere um eine Vorrichtung handeln, wie in den Wikipedia Artikeln
https://de.wikipedia.org/w/index.php?title=Sicherheitswerkbank&oldid=227074093
   und
https://en.wikipedia.org/w/index.php?title=Laminar_flow_cabinet&oldid=1142649438
   beschrieben.

Im zweiten Innenraum der Sicherheitswerkbank können dabei insbesondere eine Reinraumatmosphäre und/oder sterile Bedingungen herrschen und/oder aufrechterhalten werden, insbesondere Bedingungen gemäss Reinraumklasse ISO 14644-1.

Als Entnahmeöffnung kann insbesondere eine Arbeitsöffnung der Sicherheitswerkbank dienen, durch welche Bedienpersonal, insbesondere eine Praxisassistenz oder ein(e) Dentalhygieniker(in), in den zweiten Innenraum hineingreifen und dort Manipulationen vornehmen kann. Die Arbeitsöffnung kann mittels einer Klappe, Scheibe oder Ähnlichem verschliessbar sein. Die Arbeitsöffnung kann insbesondere so ausgestaltet sein, dass sie es einer sich vor der Sicherheitswerkbank befindlichen, insbesondere stehenden, erwachsenen Person ermöglicht, mit zumindest einer Hand sämtliche Punkte im zweiten Innenraum zu erreichen, insbesondere problemlos zu erreichen, insbesondere um dort Manipulationen vornehmen zu können.

Da die Entladeöffnung wie vorstehend beschrieben mit der Befüllungsöffnung verbunden ist, kann das Bedienpersonal Sterilisationsgut durch den zweiten Innenraum, die Befüllungsöffnung und die Entladeöffnung in den Sterilisator einbringen anschliessend die Entladeöffnung mittels der Endladeklappe verschliessen, und das Sterilisationsprogramm starten. Nach dem Ende des Sterilisationsprogramms kann das Bedienpersonal das Sterilgut durch die Entladeöffnung und die Befüllungsöffnung entnehmen und innerhalb des zweiten Innenraums platzieren, wozu insbesondere Ablagen in Form von Tablaren, Trays, Sortimentskästen oder -boxen, etc. vorgesehen sein können.

Zusätzlich zur Entladeöffnung kann der Sterilisator eine separate Beladeöffnung zum Einbringen von Sterilisationsgut in den ersten Innenraum aufweisen sowie eine Beladeklappe (131), mittels welcher die Beladeöffnung fluid- und druckdicht verschlossen werden kann. Die Beladeöffnung kann von der Entladeöffnung entfernt angeordnet sein, insbesondere an einer anderen, insbesondere gegenüberliegenden Seite des Sterilisators. Durch die separate Beladeöffnung entfällt die Notwendigkeit, Sterilisationsgut durch den zweiten Innenraum, in den Sterilisator, insbesondere den ersten Innenraum, einbringen zu müssen, wie dies bei einem Autoklaven mit einer einzigen Öffnung erforderlich ist. Damit kann eine Sicherheit der sterilen Lagerung des Sterilguts im zweiten Innenraum weiter erhöht werden. Ferner kann eine Handhabung des Systems erleichtert und/oder beschleunigt werden.

Bei einem erfindungsgemässen System kann ein Verriegelungsmechanismus vorgesehen sein, welcher dazu eingerichtet sein kann, die Entladeklappe nach einem Verschliessen zu verriegeln, so dass diese erst nach einem Ende, insbesondere einem regulären Ende, des Sterilisirungsprozess wieder geöffnet werden kann. Dies verhindert, dass versehentlich Verunreinigungen in den zweiten Innenraum gelangen können, und eine dort herrschende Sterilität und/oder Raumreinbedingungen komprimieren und/oder zunichte machen kann. Ohne einen entsprechenden Verriegelungsmechanismus kann dies insbesondere durch versehentliche Entnahme und ggf. Einlagerung nicht (ausreichend) sterilisierter Instrumente geschehen.

Bei einem System mit einem Sterilisator mit einer separaten Beladeöffnung kann der Verriegelungsmechanismus zusätzlich oder alternativ dazu eingerichtet sein, ein Öffnen der Entladeklappe zu verhindern, solange die Beladeklappe geöffnet ist, und/oder ein Öffnen der Beladeklappe zu verhindern, solange die Entladeklappe geöffnet ist. Dadurch kann verhindert werden, dass Be- und Entladeklappe gleichzeitig geöffnet sind und dadurch Verunreinigungen durch den ersten in den zweiten Innenraum gelangen können, womit wiederum eine dort herrschende Sterilität und/oder Raumreinbedingungen komprimiert und/oder zunichte gemacht werden könnte.

Ein Verriegelungsmechanismus wie vorstehend beschrieben erlaubt insbesondere eine prozesssichere Überwachung von Be- und/oder Entladeklappe.

Ein Verfahren zum Sterilisieren und sterilen Lagern medizinischer Instrumente gemäss der Erfindung wie nachfolgend beansprucht kann die Merkmale von Anspruch 1 weiter unten umfassen.

Ein erfindungsgemässes Verfahren zum Sterilisieren von medizinischen Instrumenten kann insbesondere folgenden Schritte umfassen:
a. Einbringen von Sterilisationsgut umfassend zu sterilisierende medizinische Instrumente in einen ersten Innenraum (11) eines Sterilisators (1), insbesondere eines Autoklaven;
b. Sterilisieren des Sterilisationsguts im Sterilisator, insbesondere mittels eines Sterilisierungsprozesses des Sterilisators, um Sterilgut umfassend die medizinischen Instrumente zu erhalten;
c. Entnehmen des Sterilguts durch eine Entladeöffnung (12) des Sterilisators;
d. Einbringen des Sterilguts in einen zweiten Innenraum (21) einer Sicherheitswerkbank (2), insbesondere einer Laminar Flow Box durch eine Befüllungsöffnung (23) der Sicherheitswerkbank;
e. Lagern des Sterilguts im zweiten Innenraum (21) der Sicherheitswerkbank;
f. Entnehmen des Sterilguts durch eine Entnahmeöffnung der Sicherheitswerkbank;
dadurch gekennzeichnet, dass
g. dass die Entladeöffnung mit der Befüllungsöffnung verbunden ist oder wird, insbesondere derart, dass die Entladeöffnung in die Befüllungsöffnung mündet und/oder geführt ist, insbesondere in die Befüllungsöffnung hineinragt; und
h. das Sterilgut dem zweiten Innenraum erst unmittelbar vor einem medizinischen, insbesondere zahnmedizinischen, Eingriff entnommen wird, bei welchem die medizinischen Instrumente eingesetzt werden.

Für die Verfahren gelten die weiter oben gemachten Ausführungen, insbesondere betreffend den Sterilisator und die Sicherheitswerkbank, analog.

Das Sterilisationsgut kann dabei in unverpacktem Zustand, insbesondere ohne in einen Sterilisationsbeutel eingeschweisst (worden) zu sein, in den Sterilisator eingebracht werden, und in diesem in ebenso unverpacktem Zustand sterilisiert werden. Unter einem unverpackten Zustand kann jeder Zustand verstanden werden, bei dem das Sterilisationsgut, insbesondere die einzelnen medizinischen Instrumente, nicht in einem hermetisch verschlossen Behälter verpackt sind, welcher sich insbesondere nicht zerstörungsfrei und/oder reversibel öffnen lässt. In einem derartigen unverpackten Zustand ist eine sterile Lagerung nach erfolgter Sterilisation, insbesondere in einer (zahn-)ärztliche Praxis, nicht ohne weiteres möglich, unter Anderem weil das erhaltene Sterilgut unmittelbar nach Entnahme aus einem herkömmlichen Sterilisator mit einer (kontaminierten) Umgebungsluft in Berührung kommen würde.

Um insbesondere eine einfache und effiziente Handhabung zu ermöglichen, kann das Sterilisationsgut auf Tablare, Trays oder Ähnliches gelegt werden, welche dann mit dem Sterilisationsgut in den Sterilisator eingebracht werden können, wobei die Tablare, Trays oder Ähnliches durch die Entladeöffnung in den ersten Innenraum eingebracht werden kann, insbesondere durch die Arbeitsöffnung, den zweiten Innenraum und/oder die Befüllungsöffnung hindurch. Alternativ kann das Sterilisationsgut in Kästen oder Boxen, insbesondere Sortimentskästen oder -boxen, welche verschliessbar, insbesondere wiederverschliessbar, sein können, und ebenfalls durch die Entladeöffnung in den ersten Innenraum eingebracht werden kann, insbesondere durch die Arbeitsöffnung, den zweiten Innenraum und/oder die Befüllungsöffnung hindurch.

Nach erfolgter Sterilisation kann das erhaltene Sterilgut auf den Tablaren, Trays etc. bzw. in den Boxen oder Kästen belassen werden und diese können im zweiten Innenraum (21) der Sicherheitswerkbank gelagert werden. Dazu können insbesondere geeignete Ablagen wie Regale, Ständer oder Ähnliches vorhanden sein, die dazu ausgelegt sind eine Vielzahl, insbesondere mehr als 10, vorzugsweise mehr als 50 solcher Tablare, Trays, Boxen, Kästen oder Ähnliches zu fassen. Tablare, Trays, Boxen, Kästen oder Ähnliches werden im Folgenden auch mit der Sammelbezeichnung Träger bezeichnet.

Die Träger, insbesondere Tablare, Trays oder Ähnliches können dabei jeweils nur mit einer einzigen Sorte (insbesondere untereinander identischer) medizinischer Instrumente beladen werden. Die Träger, insbesondere verschliessbare Boxen oder Kästen, können jedoch auch mit jeweils einem Sortiment von Instrumenten beladen werden, wobei ein Sortiment eine Vielzahl von verschiedenen Instrumenten in einer Anzahl benötigt, wie sie für einen bestimmten (zahn-)medizinischen Eingriff (üblicherweise) benötigt werden oder erforderlich sind. Für jeden einzelnen einer Vielzahl von denkbaren Eingriffen kann dabei ein spezifisches Sortiment vorgesehen sein, welches sich insbesondere von (allen) anderen Sortimenten für andere Eingriffe unterscheiden kann. Dazu können die Täger Bereiche und/oder Markierungen aufweisen, in welche jeweils nur (zahn-)medizinische Instrumente einer bestimmten Sorte passen. Jeder der Bereiche kann insbesondere eine Vertiefung umfassen, welche eine Form aufweist, die einem Abdruck entspricht, welchen ein in sie passendes Instrument bei einem Einpressen in ein weiches, plastisch verformbares Material hinterlassen würde. Somit kann eine Vielzahl von Arten von Trägern existieren, von denen jeder mit den üblicherweise für einen oder mehrere Arten (zahn-)medizinischer Eingriffe benötigten oder erforderlichen Instrumenten bestückt ist, von denen insbesondere jeder zur Aufnahme (genau) eines spezifischen Sortiments geeignet ist.

An den medizinischen Instrumenten und/oder den Trägern kann eine (erste) eindeutige Kennzeichnung vorgesehen sein oder werden, beispielsweise in Form eines Strichcodes, eines QR-Codes, einer Zahl oder Zeichenfolge oder Ähnlichem. Die Kennzeichnung kann untrennbar mit dem jeweiligen Instrument oder Träger verbunden sein, beispielsweise eingraviert, eingestempelt, gegossen etc. Die Kennzeichnung kann auch in einem elektronischen Bauteil gespeichert sein, welches insbesondere einen nichtvolatilen Speicher enthält, und kann über einen elektrischen Kontakt und/oder kontaktlos, beispielsweise mittels Induktion oder elektromagnetische Wellen, ausgelesen werden. Bei dem elektronischen Bauteil kann es sich insbesondere um einen RFID-Chip handeln, insbesondere um einen Transponder wie beispielsweise unter
https://de.wikipedia.org/w/index.php?title=RFID&oldid=248474675
   beschrieben. Das elektronische Bauteil kann derart mit dem Instrument oder Träger verbunden, insbesondere irreversibel verbunden, sein oder werden, dass es nicht ohne Zerstörung des Instruments bzw. Trägers oder des Bauteils selbst entfernt werden kann - oder zumindest nicht ohne ein erhebliches Risiko, insbesondere von mehr als 50%, einer solchen Zerstörung. Alternativ kann das elektronische Bauteil derart mit dem Instrument oder Träger verbunden sein, das ein spezielles Werkzeug, insbesondere nicht im Handel erhältliches Werkzeug, und/oder ein Schlüssel, ein Sicherheitsschlüssel, erforderlich ist, um die Verbindung zu lösen. Die Verbindung kann dabei insbesondere mit ein oder mehreren Sicherheitsschrauben wie beispielsweise unter
https://de.wikipedia.org/w/index.php?title=Sicherheitsschraube&oldid=252269945
   beschrieben erstellt oder gesichert werden. Denkbar ist auch ein elektronischer Verriegelungsmechanismus, der insbesondere über den RFIP-Chip gesteuert, insbesondere verriegelt und entriegelt werden kann. Dieser kann insbesondere drahtlos mit dem RFID-Chip verbunden sein, wenn letzterer mit dem Instrument oder Träger verbunden ist, oder auch im RFID-Chip integriert sein.

Das elektronische Bauteil kann einen Signalgeber umfassen, insbesondere einen optischen Signalgeber wie beispielsweise eine Leuchtdiode (LED). Das elektronische Bauteil kann einen elektrischen Energiespeicher, insbesondere eine Batterie, umfassen, der insbesondere den Signalgeber mit Energie versorgen kann.

Für jedes Instrument und/oder für jeden mit Instrumenten bestückten Träger kann ein Zeitpunkt, insbesondere ein Datum, und/oder ein Zeitraum an bzw. in welchem (letztmalig) eine Sterilisierung im Sterilisator erfolgt ist, in einer Datenbank gespeichert werden, gegebenenfalls zusammen mit weiteren Daten betreffend den entsprechenden (letzten) Sterilisierungsprozess, wie beispielsweise einem oder mehreren Prozessparametern wie insbesondere von Sensoren gemessen und/oder entsprechenden Sollwerten. Ebenfalls gespeichert werden können Informationen betreffend einen Zustand des Sterilisators, insbesondere einen Wartungsstatus, mögliche aufgetretene Warn- oder Fehlermeldungen, einen letztmaligen Einsatz des Instruments und/oder des Trägers, das Bedienpersonal, etc. Jegliche Speicherung von Daten kann vorzugsweise DSGVO-konform erfolgen. Instrument und/oder Träger werden dabei anhand ihrer eindeutigen Kennzeichnung identifiziert, die ebenfalls abgespeichert werden kann, entweder direkt oder über eine Abbildung auf ein in der Datenbank speicherbares Datenformat, wobei die Abbildung insbesondere eindeutig und/oder umkehrbar sein kann. Falls die Kennzeichnung auf einem elektronischen Bauteil gespeichert ist, kann alternativ oder zusätzlich zumindest ein Teil der vorstehend genannten Daten und/oder Informationen auch auf diesem Bauteil gespeichert werden.

Die Datenbank kann auf, in und/oder von einer Datenverarbeitungsanlage oder einem Datenverarbeitungssystem gespeichert und/oder verwaltet werden. Dabei kann es sich insbesondere um einen Computer oder ein Computersystem handeln, welches sich physisch (vollständig) in der (zahn-)ärztlichen Praxis befindet, in der das erfindungsgemässe Verfahren angewandt wird und/oder zum Einsatz kommt. Es kann sich auch um ein verteiltes Computersystem handeln, insbesondere ein Client-Server-System, bei welchem die Datenbank auf einem Server gespeichert ist, und ein Zugriff über einen oder mehrere Clients möglich ist; oder ein Cloudsystem, bei welchem ein Zugriff auf die Datenbank über ein (weltweites) Computernetz erfolgen kann, insbesondere über das Internet.

Die Datenverarbeitungsanlage kann mit dem oder den elektronischen Bauteil(en) eines oder mehrerer Instrumente und/oder Träger verbunden oder verbindbar sein, insbesondere über eine drahtlose Signal- und oder Datenverbindung. Soweit es sich beim elektronischen Bauteil um einen RFID-Chip handelt, kann die Verbindung über ein RFID-Lesegerät erfolgen, welches wiederum mit der Datenverarbeitungsanlage verbunden oder verbindbar sein kann, insbesondere über eine physische Signal- und/oder Datenverbindung; oder welches in der Datenverarbeitungsanlage integriert sein kann.

Die Datenverarbeitungsanlage kann ein Erfassungsgerät für Kennzeichnungen, insbesondere einen Barcode-Scanner, einen QR-Code Scanner und/oder ein RFID-Lesegerät, umfassen und/oder mit einem derartigen Erfassungsgerät verbunden und/oder verbindbar sein, insbesondere über eine drahtlose (Signal- und/oder Daten-)Verbindung wie WLAN oder Bluetooth. Das Erfassungsgerät kann insbesondere vom Bedienpersonal, insbesondere einer Praxisassistenz oder einem/einer Dentalhygieniker(in) bedient werden. Als Erfassungsgerät kann insbesondere ein Mobilgerät wie beispielsweise ein Smartphone oder ein Tablet(computer) dienen, welches bzw. welcher eine Kamera umfasst und durch eine geeignete Applikation gesteuert wird.

Um den Zeitpunkt und/oder den Zeitraum an bzw. in welchem (letztmalig) eine Sterilisation im Sterilisator erfolgt ist für ein Instrument und/oder für einen mit Instrumenten bestückten Träger in der Datenbank zu speichern, kann die entsprechende (erste) Kennzeichnung vor und/oder nach besagter Sterilisation mit dem Erfassungsgerät erfasst und an die Datenverarbeitungsanlage übermittelt werden. Der Sterilisator kann mit der Datenverarbeitungsanlage verbunden oder verbindbar sein, insbesondere über eine Signal- und/oder Datenverbindung, und kann dazu eingerichtet sein, Prozessparameter, insbesondere deren Ist- und/oder Sollwerte, und/oder sonstige Informationen betreffend den Sterilisierungsprozess oder den Sterilisator an die Datenverarbeitungsanlage zu übermitteln, insbesondere auf eine Anforderung durch die Datenverarbeitungsanlage hin.

Auf Basis des Zeitpunkts und/oder Zeitraums an bzw. in welchem (letztmalig) eine Sterilisation im Sterilisator erfolgt ist, kann ein Verfalldatum ermittelt oder festgelegt werden, nach welchem das jeweilige Instrument und/oder die jeweiligen Instrumente, mit denen der Träger bestückt ist bzw. wurde, nicht mehr verwendet werden sollen oder dürfen, insbesondere weil das Vorliegen des sterilen Zustands als nicht mehr sicher gewährleistet angesehen wird. Das Verfalldatum kann, ggf. zusammen mit den weiter oben genannten Daten und Informationen, in der Datenbank und/oder ggf. auf dem elektronischen Bauteil gespeichert werden. Das Verfalldatum kann durch Addieren einer vorbestimmten Zeitspanne, wie zum Beispiel einen oder mehrere Monate, zu dem Zeitpunkt oder dem Ende des Zeitraums erhalten werden. Die Zeitspanne kann für alle Instrumente und/oder Träger identisch sein.

Schliesslich kann in der Datenbank eine Information betreffend eine Position im zweiten Innenraum gespeichert werden, an welcher das Instrument, die Instrumente und/oder der Träger verbracht wurde und/oder gelagert wird. Bei der Position kann es sich insbesondere um eine Position innerhalb einer Ablage wie einem Regal, Ständer oder Ähnlichem handeln. Die Ablage kann eine Vielzahl von Fächern enthalten, die für die Aufnahme von Trägern geeignet sein können, wobei insbesondere jedes Fach dazu ausgelegt sein kann, genau einen Träger und/oder genau eine Art von Trägern aufzunehmen. Die Position bzw. ein dazugehöriges und/oder korrespondierendes Fach kann dabei insbesondere durch eine Fachnummer identifiziert werden, welche wiederum eine Position innerhalb einer definierten Reihe und/oder Spalte umfassen kann, wie beispielsweise *n*-te Reihe von oben, *m*-tes Fach von links, wobei *n* und *m* insbesondere ganze Zahlen repräsentieren können.

Zumindest an einem Teil der Positionen der Ablage, insbesondere zumindest an einem Teil der Fächer, kann eine (zweite) eindeutige Kennzeichnung vorgesehen sein oder werden, beispielsweise in Form eines Strichcodes, eines QR-Codes, einer Zahl oder Zeichenfolge oder Ähnlichem. Die Kennzeichnung kann untrennbar mit dem jeweiligen Fach verbunden sein, beispielsweise eingraviert, eingestempelt, gegossen etc. Die Kennzeichnung kann auch in einem elektronischen Bauteil gespeichert sein, wie weiter oben bereits beschrieben. Die Speicherung der Information betreffend die Position im zweiten Innenraum in der Datenbank kann durch Erfassen der zugehörigen (zweiten) eindeutigen Kennzeichnung mit dem Erfassungsgerät erfolgen, vorzugsweise unmittelbar bevor und/oder unmittelbar nachdem ein oder mehrere Instrumente und/oder ein Träger an die Position, insbesondere in das Fach, verbracht wird bzw. wurde.

Alternativ oder zusätzlich kann an zumindest an einem Teil der Positionen, insbesondere Fächer, ein (zweites) RFID-Lesegerät vorgesehen, insbesondere fest installiert sein, welches mit der Datenverarbeitungsanlage verbunden und/oder verbindbar sein, insbesondere über eine physische oder eine drahtlose (Signal- und/oder Daten-)Verbindung. Die entsprechenden RFID-Lesegeräte können dazu ausgelegt sein, die auf einem oder mehreren RFID-Chips gespeicherten Kennzeichnungen eines an der Position, insbesondere im zugehörigen Fach gelagerten Trägers und/oder darin befindlicher Instrumente auszulesen und an die Datenverarbeitungsanlage zu übermitteln, welche sie insbesondere in der Datenbank speichern bzw. mit der Position bzw. dem Fach verknüpfen kann. Das Auslesen der Kennzeichnungen kann dabei automatisiert ausgelöst werden, beispielsweise mittels eines Annäherungssensors, wenn ein Instrument und/oder Träger in ein Fach verbracht und/oder einem Fach entnommen wird.

Die Ablage kann einen oder mehrere Signalgeber umfassen, insbesondere optische Signalgeber wie beispielsweise Leuchtdioden (LEDs), mittels welcher eine oder mehrere Positionen angezeigt und/oder identifiziert werden können, insbesondere als Reaktion auf ein entsprechendes Signal von der Datenverarbeitungsanlage. Insbesondere kann für jedes Fach und/oder an jedem Fach ein Signalgeber vorgesehen sein.

Für einen oder mehrere mit Instrumenten bestückte Träger kann in der Datenbank ferner gespeichert sein bzw. werden, für welche Art von Eingriff, insbesondere für welchen oder für welche bestimmten (zahn-)medizinischen Eingriff(e) der Träger die (üblicherweise) benötigten Instrumente enthalten sind. Diese Information kann alternativ oder zusätzlich anhand der Kennzeichnung ersichtlich und/oder ermittelbar sein, beispielsweise anhand eines Buchstabens oder einer Ziffer an einer vorgegebenen Stelle der als Kennzeichnung verwendeten Zeichenfolge. Falls die Kennzeichnung auf einem elektronischen Bauteil gespeichert ist, kann besagte Information ebenfalls auf besagtem Bauteil gespeichert (und abrufbar) sein.

Wenn ein Instrument und/oder ein Träger aus dem zweiten Innenraum der Sicherheitswerkbank mit der Absicht entnommen wird oder entnommen werden soll, einen bestimmten und/oder geplanten Eingriff an einem bestimmten Patienten vorzunehmen, kann zunächst anhand einer Abfrage der Datenbank unter Verwendung der an Instrument oder Träger angebrachten Kennzeichnung sichergestellt werden, dass das Verfalldatum für dieses Instrument und/oder diesen Träger, insbesondere die Instrumente, mit welchen dieser Träger bestückt ist, noch nicht überschritten wurde und/oder in der Zukunft liegt.

Ferner kann überprüft werden, ob ein gleichartiges Instrument oder ein gleichartiger, insbesondere gleich bzw. mit gleichen Instrumenten bestückter, Träger vorrätig ist, insbesondere sich im zweiten Innenraum der Sicherheitswerkbank befindet und/oder gelagert wird, dessen Verfalldatum weniger weit in der Zukunft liegt, insbesondere mehrere Tage weniger weit. Ist dies der Fall, kann besagtes gleichartiges Instrument oder besagter gleichartiger Träger anhand der eindeutigen Kennzeichnung lokalisiert und/oder identifiziert werden, und/oder anstelle des zuvor vorgesehenen Instruments oder Trägers zur Entnahme vorgesehen und/oder entnommen werden. Eine entsprechende Überprüfung kann so lange und/oder so oft wiederholt werden, bis kein Instrument oder Träger mit einem (noch) älteren bzw. weniger weit in der Zukunft liegenden Verfalldatum mehr gefunden bzw. ermittelt wird. Somit kann zumindest für einzelne Arten von Trägern und/oder Instrumenten eine Art sogenanntes "first in, first out" (FIFO) Prinzip implementiert werden.

Durch die vorstehend beschriebene Herangehensweise kann effizient verhindert werden, dass für Instrumente und/oder Träger in grösserer Zahl das Verfalldatum überschritten wird, ohne dass diese benutzt wurden - oder selbigem zumindest entgegengewirkt werden.

Das gefundene, insbesondere zuletzt gefundene, Instrument oder der gefundene, insbesondere zuletzt gefundene, Träger kann dann wiederum anhand der eindeutigen Kennzeichnung lokalisiert und/oder identifiziert werden, und schliesslich dem zweiten Innenraum der Sicherheitswerkbank entnommen werden, um den bestimmten Eingriff durchzuführen. Soweit in der Datenbank Information betreffend eine Position der Instrumente oder Träger im weiten Innenraum der Sicherheitswerkbank gespeichert wurden oder sind, können diese zum Lokalisieren des gefundenen, insbesondere zuletzt gefundenen, Instruments bzw. Trägers herangezogen werden. Soweit die Sicherheitswerkbank eine Ablage mit Signalgeber(n) wie vorstehend beschrieben umfasst, können diese dabei auf ein Signal von der Datenverarbeitungsanlage hin die Position des gefundenen, insbesondere zuletzt gefundenen, Instruments bzw. Trägers signalisieren bzw. anzeigen.

Ist die Kennzeichnung auf einem elektronischen Bauteil gespeichert, welches zudem einen Signalgeber umfasst, kann dieser veranlasst werden, ein Signal, insbesondere ein optisches Signal, auszusenden, um die Lokalisierung zu erleichtern. Die besagte Veranlassung kann durch die Datenverarbeitungsanlage erfolgen, die beispielsweise wie oben beschrieben über ein RFID-Lesegerät mit dem elektronischen Bauteil verbunden oder verbindbar sein kann.

Auf diese Weise kann sichergestellt werden, dass jeweils dasjenige Instrument oder derjenige Träger einer Art zum Einsatz kommt, dessen Verfalldatum als nächstes erreicht wird, wodurch eine Anzahl von Instrumenten und/oder Trägern, deren Verfalldatum überschritten wird, minimiert werden kann. Dies erlaubt insbesondere eine Reduzierung und/oder Minimierung von Aufwand und Ressourcen wie insbesondere Energie.

Vor, während oder unmittelbar nach dem geplanten Eingriff kann die eindeutige Kennzeichnung und/oder die für das zugehörige Instrument oder den zugehörigen Träger in der Datenbank gespeicherten Daten oder Informationen, insbesondere betreffend den letzten Sterilisierungsprozess, zumindest teilweise in eine (elektronische) Patientenakte oder ein (elektronisches) Patientendossier übertragen oder kopiert werden, welche dem Patienten zugeordnet ist, an welchem der Eingriff durchgeführt werden soll, wird oder wurde.

Die (elektronische) Patientenakte oder das (elektronische) Patientendossier kann dabei auf derselben Datenverarbeitungsanlage oder demselben Datenverarbeitungssystem gespeichert sein wie die Datenbank. Alternativ kann die (elektronische) Patientenakte oder das (elektronische) Patientendossier auf einer zweiten Datenverarbeitungsanlage oder an einem zweiten Datenverarbeitungssystem gespeichert sein, an welche die (erste) Datenverarbeitungsanlage oder das (erste) Datenverarbeitungssystem Daten und/oder Informationen übertragen kann, beispielsweise über ein Kommunikationsnetzwerk wie beispielsweise ein (geschlossenes) TCP/IP-Netzwerk und/oder das Internet.

Vorteilhafte Ausgestaltungen der Erfindung sind Gegenstand der Unteransprüche, und/oder ergeben sich aus der nachfolgenden Beschreibung in Verbindung mit den Zeichnungen.

Im Folgenden wird anhand beiliegender Zeichnungen ein bevorzugtes Ausführungsbeispiel der Erfindung beschrieben.

Es zeigen
- Figur 1: schematisch einen grundsätzlichen Aufbau eines erfindungsgemässen Systems zum Sterilisieren und sterilen Lagern medizinischer Instrumente;
- Figuren 2a-c: Details betreffend die Sicherheitswerkbank.

### Wege zur Ausführung der Erfindung

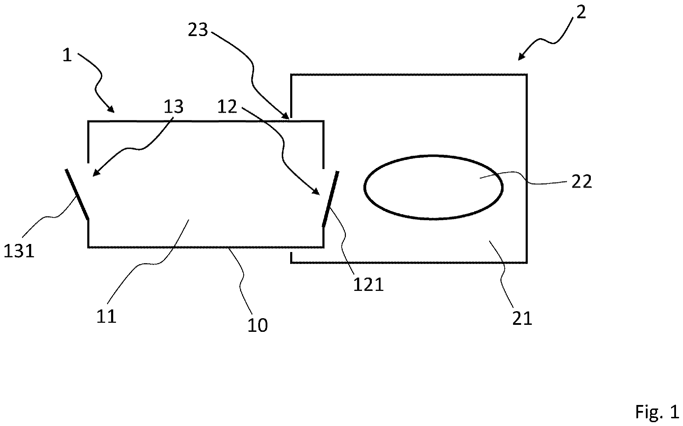

Figur 1 zeigt eine schematische Darstellung eines erfindungsgemässen Systems und illustriert das erfindungsgemässe Bereitstellungsverfahren. Definitionen der Bezugszeichen finden sich in den Patentansprüchen, wo diese in Klammern angegeben sind. Ein Teil des Sterilisators 1 mit einem ersten Innenraum 11 ist bzw. wird durch eine Befüllungsöffnung 23 einer Sicherheitswerkbank 2 teilweise in einen zweiten Innenraum 21 besagter Sicherheitswerkbank eingeschoben, so dass eine Entladeöffnung 12 des Sterilisators in die Befüllungsöffnung mündet, insbesondere in die Befüllungsöffnung hineinragt. Eine erste Form der Befüllungsöffnung 23 ist dabei vorzugsweise so auf eine zweite Form eines Gehäuses 10 des Sterilisators abgestimmt, dass zwischen besagtem Gehäuse und einem Rand der Befüllungsöffnung 23 lediglich ein schmaler Spalt verbleibt, dessen Breite vorzugsweise an keiner Stelle grösser ist als wenige Millimeter. Der Spalt kann vorzugsweise fluiddicht abgedichtet sein oder werden, insbesondere mittels einer Silikonfuge oder einer Gummidichtung. Durch den schmalen Spalt und insbesondere eine Abdichtung kann verhindert werden, dass Verunreinigungen durch die Befüllungsöffnung in den zweiten Innenraum gelangen können, womit eine dort herrschende Sterilität und/oder Raumreinbedingungen komprimiert und/oder zunichte gemacht werden könnte.

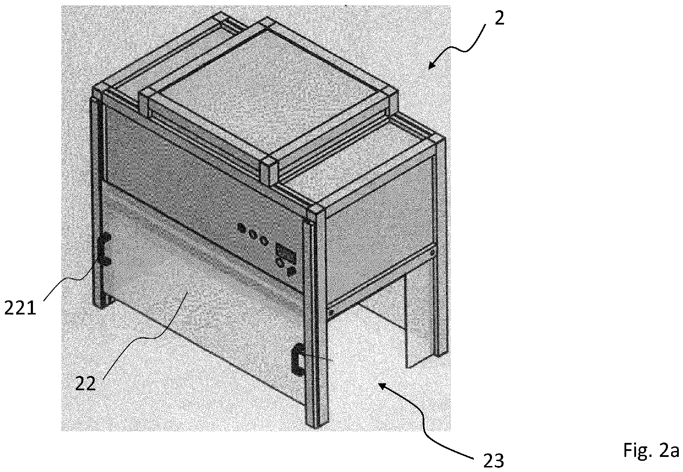

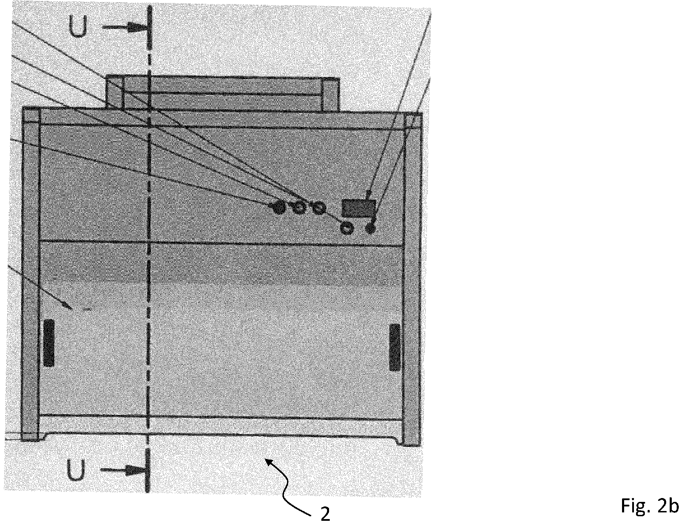

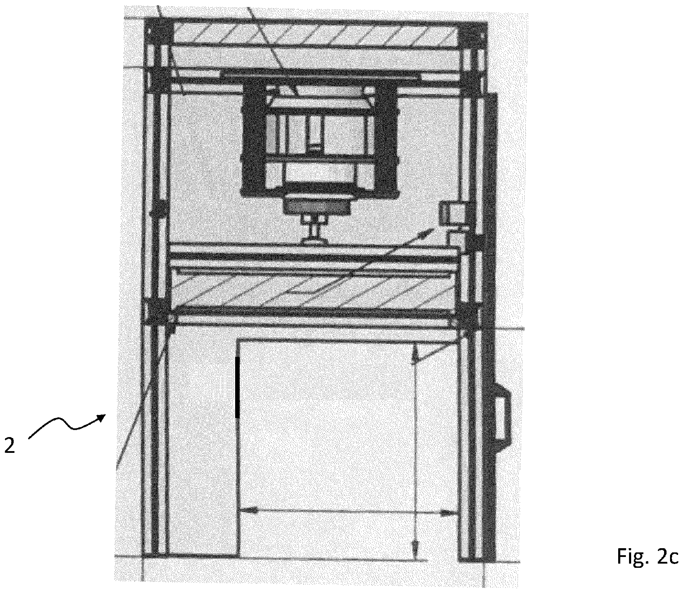

Die Figuren 2a bis 2c zeigen Details einer exemplarischen Sicherheitswerkbank 2 zur Verwendung in einem System oder Verfahren wie vorstehend beschrieben und oder nachfolgend beansprucht. In die Sicherheitswerkbank in Form einer Laminarflow-Kabine (LA) gebaut nach Reinraumklassen ISO 14644-1 wird seitlich durch eine Aussparung, welche als Befüllungsöffnung 23 dient, ein Autoklav von der TürSeite her, insbesondere mit derjenigen Seite, an welcher die Entladeklappe vorgesehen ist, zumindest annähernd 10 cm weit eingeschoben und bleibt so installiert. Die Masse des, für diesen Fall, vorgesehen Autoklavs sind zumindest annähernd B x H x L 450 x 440 x 620 mm. Alternativ wäre es auch möglich, die Befüllungsöffnung an einer hinteren Wand der LA vorzusehen und entsprechend den Autoklaven von der hinteren Wand her einzubauen, dies erfordert jedoch mehr Tiefe für eine Aufstellung eines entsprechenden Systems.

Sobald der Entkeimungsvorgang, insbesondere der Sterilisationsprozess, beendet ist und sich die Türe des Autoklavs automatisch öffnet, bleibt der Inhalt in Reinraumatmosphäre.

Das Personal kann dann nach Anweisung des Arztes den Inhalt, also die Werkzeuge und Instrumente etc. in die Trays für den Arzt oder DA einordnen und unter Reinraumbedingungen so lange lagern, bis ein Patient auf dem Stuhl sitzt. Dann werden die Trays mit einem Deckel an den Arbeitsplatz gebracht. Der Deckel wird erst entfernt, wenn die Werkzeuge oder die Instrumente etc. gebraucht werden. Sobald die Behandlung abgeschlossen ist, werden die Instrumente etc. mit dem Tray und dem Deckel in die Reinigung zurückgestellt, insbesondere eine sog. rote Zone, in welcher Instrumente aufbewahrt werden, die vor einem neuerlichen Einsatz zunächst sterilisiert werden müssen.

Nach der Reinigung der Instrumente etc. beginnt der Prozess von Neuem.

Alle Teile, wie die Lagergestelle, die Trays und deren Deckel, welche sich unter dem LA befinden, lassen sich im Autoklav entkeimen. Das soll periodisch gemäss dem Ausdruck vom Autoklav oder nach Anweisung des Arztes durchgeführt werden.

Die jetzige Lösung soll sich für Praxen bis zwei Zahnarztplätze und bis zu zwei DH-Arbeitsplätze eignen.

Es sind 3 x 7 Stapelplätze für das Tray Format mit Deckel B x H x L 186 x 45 x 288 mm vorgesehen.

Eine Arbeitsöffnung 22 der LA ist mittels einer Frontscheibe 221 verschliessbar, welche insbesondere vertikal verschiebbar sein kann, um die Arbeitsöffnung je nach Position besagter Frontscheibe freizugeben oder zu verschliessen.

Die exemplarische Sicherheitswerkbank weist einen G4-Vorfilter auf, der insbesondere der Prüfnorm ISO 16890 entspricht mit einer Filterklasse ISO coarse 85% / G4; ferner einen H1-Schwebstoffiler, der insbesondere der Prüfnorm EN 1822 entspricht, insbesondere mit einer Filterklasse H14, einem minimalen Abscheidegrad von zumindest annähernd 99,995% und/oder einem maximalen Durchlassgrad von zumindest annähernd 0,00%.

Im ersten Innenraum der Sicherheitswerkbank kann eine Beleuchtung vorgesehen sein, welche insbesondere LEDs umfassen kann.

Für grössere Praxen können die Dimensionen des LA und ev. des Autoklavs sowie die Stapelplätze angepasst werden.

Weiter ist ein Autoklav denkbar, welcher sich von aussen beladen lässt, insbesondere über eine separate Beladeöffnung, und sich dieser nach dem Sterilisierungsvorgang innen öffnet. Beide Türen müssen prozesssicher überwacht werden. Das hat den Vorteil, dass nie unsteriles Material in die Reinraumatmosphäre gelangt. Zudem ist das Handling einfacher.

Sofern nicht anders angegeben, kann ein fluid- und/oder druckdichtes Element, insbesondere ein Behälter, ein Volumen oder ein Rückhaltevolumen, ein Fluid, insbesondere eine Flüssigkeit oder ein Gas, speichern, enthalten, vorrätig halten und/oder bevorraten, ohne dass das Fluid aus besagtem Element austritt oder austreten kann.

Sofern nicht anders angegeben, kann eine fluid- und/oder druckdichte Verbindung zwischen zwei ein Fluid, insbesondere eine Flüssigkeit oder ein Gas, leitenden, führenden, bevorratenden und/oder speichernden Elementen wie insbesondere Leitungen, Rohren, Behältern, Volumen, Rückhaltevolumen, etc., eine Verbindung bezeichnen, durch welche Fluid verlustfrei, insbesondere ohne durch die oder im Bereich der Verbindung auszutreten, von einem Element zum anderen strömen und/oder geleitet werden kann, insbesondere unter den üblicherweise, vor allem in einem Betriebszustand einer die Elemente umfassenden Apparatur, herrschenden, statischen und/oder dynamischen Fluiddrücken. In analoger Weise kann ein fluid- und/oder druckdichter Verschluss oder eine fluid- und/oder druckdichte (Ab-)dichtung ein Element, insbesondere eine Öffnung eines Elements, verschliessen, ohne dass Fluid durch den oder im Bereich des Verschlusses bzw. der (Ab-)dichtung austreten kann. Eine Fluidverbindung kann insbesondere eine fluid- und/oder druckdichte Verbindung sein. Aus einem oder in ein fluid- und/oder druckdicht verschlossenes Volumen, insbesondere aus einem oder in einen fluid- und/oder druckdicht verschlossenen Behälter, kann. Ein druckdichtes Element, eine druckdichte Verbindung oder ein druckdichter Verschluss bleibt dabei auch unter einer wesentlichen Druckdifferenz, insbesondere zwischen einem Inneren und einem Äusseren des Elements oder der Verbindung oder beiden Seiten des Verschlusses, fluiddicht. Eine wesentliche Druckdifferenz kann mindestens einen Faktor 1,5, 2,0 oder 5,0 bedeuten, wenn sich sowohl im Inneren als auch dem Äusseren des Elements oder der Verbindung oder auf beiden Seiten des Verschlusses ein gasförmiges Fluid vorliegt. Eine wesentliche Druckdifferenz kann mindestens einen Faktor 10, 50 oder 200 bedeuten, wenn im Inneren oder Äusseren des Elements oder der Verbindung oder auf mindestens einer Seiten des Verschlusses ein Fluid in Form einer Flüssigkeit vorliegt.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Um die Erfindung nicht zu verklären, können in gewissen Fällen wohlbekannte Strukturen und Techniken nicht im Detail gezeigt und beschrieben sein. Es versteht sich, dass Fachleute Änderungen und Abwandlungen machen können, ohne den Umfang der folgenden Ansprüche zu verlassen. Insbesondere deckt die vorliegende Erfindung weitere Ausführungsbeispiele mit irgendwelchen Kombinationen von Merkmalen ab, die von den explizit beschriebenen Merkmalskombinationen abweichen können.

Die vorliegende Offenbarung umfasst auch Ausführungsformen mit jeglicher Kombination von Merkmalen, die vorstehend oder nachfolgend zu verschiedenen Ausführungsformen genannt oder gezeigt sind. Sie umfasst ebenfalls einzelne Merkmale in den Figuren, auch wenn sie dort im Zusammenhang mit anderen Merkmalen gezeigt sind und/oder vorstehend oder nachfolgend nicht genannt sind. Auch können die in den Figuren und der Beschreibung beschriebenen Alternativen von Ausführungsformen und einzelne Alternativen deren Merkmale vom Erfindungsgegenstand beziehungsweise von den offenbarten Gegenständen ausgeschlossen sein. Die Offenbarung umfasst Ausführungsformen, die ausschliesslich die in den Ansprüchen beziehungsweise in den Ausführungsbeispielen beschriebenen Merkmale umfasst sowie auch solche, die zusätzliche andere Merkmale umfassen.

Im Weiteren schliesst der Ausdruck "umfassen" und oder Ableitungen davon andere Elemente oder Schritte nicht aus. Ebenfalls schliesst der unbestimmte Artikel "ein" bzw. "eine" und Ableitungen davon eine Vielzahl nicht aus. Die Funktionen mehrerer in den Ansprüchen aufgeführter Merkmale können durch eine Einheit beziehungsweise einen Schritt erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Die Begriffe "etwa" und "ungefähr" im Zusammenhang mit einem gegebenen Zahlenwert oder -bereich kann sich auf einen Wert beziehungsweise Bereich beziehen, der innerhalb 20%, innerhalb 10%, innerhalb 5% oder innerhalb 2% des gegebenen Werts beziehungsweise Bereichs liegt. Alle Bezugszeichen in den Ansprüchen sind nicht als den Umfang der Ansprüche einschränkend zu verstehen. Eine Angabe, wonach *a* ≈ *b* gilt, kann dahingehend zu verstehen sein, dass |*a-b*|/(|*a*|+|*b*|) < 0,2, vorzugsweise |*a-b*|/(|*a*|+|*b*|) < 0,05, höchst vorzugsweise |*a-b*|/(|*a*|+|*b*|) < 0,01 gilt, wobei a und b beliebige, an irgendeiner Stelle in diesem Dokument definierte und/oder beschriebene oder anderweitig dem Fachmann bekannte Variablen oder Grössen repräsentieren kann. Der Ausdruck «einige» kann eine Anzahl zwischen 3 und 10, vorzugsweise zwischen 5 und 9 bezeichnen.

Dass ein Merkmal oder eine Eigenschaft, beispielsweise eine spezifische, insbesondere geometrische, Form, zumindest näherungsweise ausgebildet, vorgesehen oder vorhanden ist, kann insbesondere bedeuten, dass Fertigungsvorgaben existieren, welche eine Vorgabe vorsehen, gemäss welcher das Merkmal entsprechend ausgebildet wird, wobei im Rahmen üblicher, dem Fachmann bekannter Fertigungstoleranzen eine Abweichung von der Vorgabe resultieren kann.

Dass ein Element oder Merkmal in einer Richtung ausgedehnt ist oder sich in einer Richtung erstreckt, kann insbesondere bedeuten, dass Abmessungen des Elements oder Merkmals in dieser Richtung grösser sind als in anderen, insbesondere allen anderen Richtungen, insbesondere orthogonalen Richtungen.

Die Begriffe "oben", "unten", "vorne", "hinten" beziehen sich insbesondere auf die bzw. den Aufbau des erfindungsmässen Systems in derjenigen Orientierung, in welcher dieses in Fig. 1 gezeigt ist. Dabei verläuft die *y*-Richtung von «oben» nach «unten». Eine seitliche Richtung entspricht der *z*-Richtung (wie in Fig. 1 angezeigt senkrecht zur Zeichenebene in diese hinein) oder -*z*-Richtung.

Sämtliche oben in Bezug genommenen Dokumente, insbesondere Wikipedia-Artikel, gelten als in vollem Umfang per Verweisung inkludiert.

Die Erfindung, insbesondere wie vorstehend beschrieben und/oder nachfolgend beansprucht, kann insbesondere in Form einer oder mehrerer der nachfolgend wiedergegebenen, nummerierten Ausführungsbeispiele:
1. Verfahren zum Sterilisieren von medizinischen Instrumenten, umfassend die Schritte
   a. Einbringen von Sterilisationsgut umfassend zu sterilisierende medizinische Instrumente in einen ersten Innenraum (11) eines Sterilisators (1), insbesondere eines Autoklaven;
   b. Sterilisieren des Sterilisationsguts im Sterilisator, insbesondere mittels eines Sterilisierungsprozesses des Sterilisators, um Sterilgut umfassend die medizinischen Instrumente zu erhalten;
   c. Entnehmen des Sterilguts durch eine Entladeöffnung (12) des Sterilisators;
   d. Einbringen des Sterilguts in einen zweiten Innenraum (21) einer Sicherheitswerkbank (2), insbesondere einer Laminar Flow Box durch eine Befüllungsöffnung (23) der Sicherheitswerkbank;
   e. Lagern des Sterilguts im zweiten Innenraum (21) der Sicherheitswerkbank;
   f. Entnehmen des Sterilguts durch eine Entnahmeöffnung der Sicherheitswerkbank;
   dadurch gekennzeichnet, dass
   g. dass die Entladeöffnung mit der Befüllungsöffnung verbunden ist oder wird, insbesondere derart, dass die Entladeöffnung in die Befüllungsöffnung mündet und/oder geführt ist, insbesondere in die Befüllungsöffnung hineinragt; und
   h. das Sterilgut dem zweiten Innenraum erst unmittelbar vor einem medizinischen, insbesondere zahnmedizinischen, Eingriff entnommen wird, bei welchem die medizinischen Instrumente eingesetzt werden.
2. Verfahren nach Ausführungsbeispiel 1, dadurch gekennzeichnet, dass das Sterilgut dem zweiten Innenraum höchstens zwei Stunden, vorzugsweise höchstens 30 Minuten, vor dem medizinischen, insbesondere zahnmedizinischen, Eingriff entnommen wird, bei welchem die medizinischen Instrumente eingesetzt werden.
3. Verfahren nach Ausführungsbeispiel 1 oder 2, dadurch gekennzeichnet, dass die Entladeöffnung fluiddicht, insbesondere druckdicht, mit der Befüllungsöffnung verbunden ist oder wird.
4. Verfahren nach einem der vorangehenden Ausführungsbeispiele, dadurch gekennzeichnet, dass die Entnahmeöffnung als Arbeitsöffnung (22) ausgeführt ist, und das Sterilgut dem ersten Innenraum entnommen wird, indem Bedienpersonal durch die Arbeitsöffnung, den zweiten Innenraum und die Entladeöffnung in den ersten Innenraum greift und das Sterilgut zumindest teilweise und/oder stückweise aufnimmt.
5. Verfahren nach einem der Ausführungsbeispiele 1 bis 3, dadurch gekennzeichnet, dass die Entnahmeöffnung als Arbeitsöffnung (22) ausgeführt ist, und das Sterilgut dem ersten Innenraum entnommen wird, indem Bedienpersonal durch die Arbeitsöffnung, den zweiten Innenraum, **die Befüllungsöffnung** und die Entladeöffnung in den ersten Innenraum greift und das Sterilgut zumindest teilweise und/oder stückweise aufnimmt.
6. Verfahren nach einem der vorangehenden Ausführungsbeispiele, dadurch gekennzeichnet, dass das Einbringen des Sterilisationsgut durch eine Beladeöffnung (13) in den ersten Innenraum erfolgt, welche am Sterilisator von der Entladeöffnung entfernt angeordnet ist, vorzugsweise an einer anderen, insbesondere gegenüberliegenden Seite des Sterilisators; wobei am Sterilisator insbesondere eine Beladeklappe (131) vorgesehen ist oder wird, mittels welcher die Beladeöffnung fluid- und druckdicht verschlossen werden kann.
7. Verfahren nach Ausführungsbeispiel 6, dadurch gekennzeichnet, dass
   a. die Entladeöffnung (12) vor dem Einbringen des Sterilisationsguts mittels einer Entladeklappe (121) verschlossen wird,
   b. die Entladeklappe verriegelt wird, insbesondere mittels eines Verriegelungsmechanismus, wobei
   c. die Entladeöffnung erst nach einem Ende des Sterilisierungssprozesses wieder freigegeben wird;
   d. wobei vorzugsweise die Beladeöffnung verschlossen ist oder wird, bevor die Entladeöffnung verschlossen wird und/oder die Beladeöffnung erst geöffnet wir, nachdem die Entladeöffnung verschlossen wurde.
8. Verfahren nach Ausführungsbeispiel 6 oder 7, dadurch gekennzeichnet, dass ein Verriegelungsmechanismus vorgesehen ist oder wird, der dazu eingerichtet ist, ein Öffnen der Entladeklappe zu verhindern, solange die Beladeklappe geöffnet ist, und/oder ein Öffnen der Beladeklappe zu verhindern, solange die Entladeklappe geöffnet ist.
9. Verfahren nach einem der Ausführungsbeispiele 1 bis 5, dadurch gekennzeichnet, dass
   a. das Sterilisationsgut durch die Entladeöffnung (12) in den ersten Innenraum eingebracht wird, insbesondere durch die Arbeitsöffnung, den zweiten Innenraum und die Befüllungsöffnung hindurch.
10. Verfahren nach einem der Ausführungsbeispiele 1 bis 5 oder nach Ausführungsbeispiel 9, dadurch gekennzeichnet, dass
   a. die Entladeöffnung (12) nach dem Einbringen des Sterilisationsguts mittels einer Entladeklappe (121) verschlossen wird,
   b. die Entladeklappe verriegelt wird, insbesondere mittels eines Verriegelungsmechanismus, wobei
   c. die Entladeöffnung erst nach einem Ende des Sterilisierungssprozesses wieder freigegeben wird.
11. Verfahren nach einem der vorangehenden Ausführungsbeispiele, dadurch gekennzeichnet, dass die Entladeöffnung (12) mittels einer Entladeklappe (121) verschlossen und besagte Entladeklappe derart verriegelt wird, insbesondere mittels eines Verriegelungsmechanismus, dass sie erst nach einem Ende des Sterilisationsprozesses wieder geöffnet werden kann.
12. Verfahren nach einem der Ausführungsbeispiele 6 bis 8, dadurch gekennzeichnet, dass die Beladeöffnung (13) mittels einer Beladeklappe (131) verschlossen und besagte Beladeklappe derart verriegelt wird, insbesondere mittels eines Verriegelungsmechanismus, dass sie erst nach einem Ende des Sterilisationsprozesses wieder geöffnet werden kann.
13. Verfahren nach einem der vorangehenden Ausführungsbeispiele, dadurch gekennzeichnet, dass das Sterilisationsgut, insbesondere das gesamte Sterilisationsgut, in unverpacktem, insbesondere nicht eingeschweisstem Zustand, in den ersten Innenraum eingebracht wird, wobei das Sterilisationsgut insbesondere kritische Instrumente umfasst.
14. Verfahren nach einem der vorangehenden Ausführungsbeispiele, dadurch gekennzeichnet, dass das Sterilisationsgut auf oder in einem oder mehreren Trägern, insbesondere in Form von (wieder-)verschliessbaren Boxen, in den ersten Innenraum eingebracht wird, wobei insbesondere eine Vielzahl von unterschiedlichen Trägern und/oder Trägern unterschiedlicher Art vorhanden sind oder verwendet werden, welche sich insbesondere dadurch unterscheiden, dass sie dazu ausgelegt sind, ein unterschiedliches Sortiment an medizinischen Instrumenten aufzunehmen, wobei insbesondere eine erste Art von Träger dazu ausgelegt ist, ein erstes Sortiment aufzunehmen, und eine zweite Art von Träger dazu ausgelegt ist, ein zweites Sortiment aufzunehmen, welches sich von dem ersten Sortiment unterscheidet.
15. Verfahren nach dem vorangehenden Ausführungsbeispiel, dadurch gekennzeichnet, dass
   a. das Sterilisationsgut auf oder in dem einen oder mehreren Trägern sterilisiert wird,
   b. auf oder in dem einen oder mehreren Trägern dem Sterilisator als Sterilgut entnommen wird,
   c. das Sterilgut auf oder in dem einem oder mehreren Trägern im zweiten Innenraum (21) der Sicherheitswerkbank gelagert wird; und/oder
   d. das Sterilgut auf oder in dem einem oder mehreren Trägern dem zweiten Innenraum entnommen wird.
16. Verfahren nach einem der vorangehenden Ausführungsbeispiele, dadurch gekennzeichnet, dass zumindest ein Teil der medizinischen Instrumente und/oder ein Teil der Träger eine eindeutige Kennzeichnung aufweist, welche es insbesondere erlaubt, jedes Instrument und jeden Träger von jedem anderen Instrument bzw. jedem anderen Träger zu unterscheiden, wobei das Verfahren insbesondere den Schritt eines Anbringens der eindeutigen Kennzeichnung an einem oder mehreren Instrumenten oder Trägern umfasst.
17. Verfahren nach einem der vorangehenden Ausführungsbeispiele, dadurch gekennzeichnet, dass zumindest für einen Teil der medizinischen Instrumente mit einer eindeutigen Kennzeichnung und/oder einen Teil der Träger mit einer eindeutigen Kennzeichnung ein Zeitpunkt, insbesondere ein Datum, und/oder ein Zeitraum an bzw. in welchem letztmalig eine Sterilisation im Sterilisator erfolgt ist, in einer Datenbank der entsprechenden eindeutigen Kennzeichnung zugeordnet und/oder gespeichert wird, gegebenenfalls zusammen mit weiteren Daten betreffend einen entsprechenden Sterilisierungsprozess, insbesondere den Sterilisierungsprozess gemäss Merkmal 1.b) von Ausführungsbeispiel 1.
18. Verfahren nach dem vorangehenden Ausführungsbeispiel, dass zumindest für den Teil der medizinischen Instrumente mit eindeutiger Kennzeichnung und/oder den Teil der Träger mit eindeutiger Kennzeichnung Informationen betreffend die Positionen gespeichert werden, an welchen die Instrumente oder Träger im zweiten Innenraum gelagert werden; insbesondere für mindestens ein Instrument oder für mindestens einen Träger gespeichert wird, in welchem Fach eines im zweiten Innenraum vorgesehenen Regals oder Gestells die Lagerung erfolgt.
19. Verfahren nach einem der vorangehenden Ausführungsbeispiele, dadurch gekennzeichnet, dass vor, während oder unmittelbar nach einem Eingriff die eindeutige Kennzeichnung eines verwendeten oder zur Verwendung vorgesehenen Instruments oder mit Instrumenten bestückten Trägers und/oder die für das zugehörige Instrument oder den zugehörigen Träger in der Datenbank gespeicherten Daten oder Informationen, insbesondere betreffend den letzten Sterilisierungsprozess, zumindest teilweise in eine Patientenakte oder ein Patientendossier übertragen oder kopiert werden, welche(s) dem Patienten zugeordnet ist, an welchem der Eingriff durchgeführt werden soll, wird oder wurde, insbesondere in eine elektronische Patientenakte oder ein elektronisches Patientendossier.
20. Verfahren nach einem der vorangehenden Ausführungsbeispiele, dadurch gekennzeichnet, dass vor einem Eingriff, insbesondere vor einem geplanten Eingriff, anhand der eindeutigen Kennzeichnung eines für eine Verwendung vorgesehenen Instruments oder mit Instrumenten bestückten Trägers anhand von in der Datenbank gespeicherten Daten und Informationen überprüft wird, dass ein Verfalldatum für dieses Instrument und/oder diesen Träger, insbesondere die Instrumente, mit welchen dieser Träger bestückt ist, noch nicht überschritten wurde und/oder in der Zukunft liegt.
21. Verfahren nach einem der vorangehenden Ausführungsbeispiele, dadurch gekennzeichnet, dass vor, während oder unmittelbar nach der Entnahme eines ersten Instruments oder mit Instrumenten bestückten ersten Trägers anhand von in der Datenbank gespeicherten Daten und Informationen ermittelt wird, ob ein gleichartiges zweites Instrument bzw. ein mit gleichen Instrumenten bestückter zweiter Träger existiert, insbesondere im zweiten Innenraum der Sicherheitswerkbank gelagert wird, für welches bzw. für welchen ein Verfalldatum weniger weit in der Zukunft liegt als ein Verfalldatum für das erste Instrument bzw. für den ersten Träger, insbesondere mindestens mehrere Tage weniger weit in der Zukunft.
22. Verfahren nach dem vorangehenden Ausführungsbeispiel, dadurch gekennzeichnet, dass die in diesem Ausführungsbeispiel beschriebenen Schritte so oft bzw. so lange wiederholt werden, bis kein Instrument oder Träger mehr ermittelt wird, für welches bzw. für welchen das Verfalldatum weniger weit in der Zukunft liegt als für das zuletzt ermittelte Instrument bzw. den zuletzt ermittelten Träger.
23. Verfahren nach einem der vorangehenden Ausführungsbeispiele, dadurch gekennzeichnet, dass
   a. vor einem geplanten Eingriff und/oder während eines Eingriffs bestimmt wird, welche Art von Instrument und/oder welche Art von mit Instrumenten bestücktem Träger benötigt wird,
   b. anhand von in der Datenbank gespeicherten Daten und Informationen ein Instrument und/oder ein Träger der bestimmten Art ermittelt wird, für welchen ein Verfalldatum am wenigsten weit in der Zukunft liegt;
   c. das ermittelte Instrument oder der ermittelte Träger dem zweiten Innenraum entnommen wird, um den Eingriff durchzuführen.
24. Verfahren nach dem vorangehenden Ausführungsbeispiel, wobei das ermittelte Instrument oder der (zuletzt) ermittelte Träger anhand er eindeutigen Kennzeichnung, die es oder er aufweist, identifiziert oder lokalisiert wird.
25. Verfahren nach einem der vorangehenden Ausführungsbeispiele, dadurch gekennzeichnet, dass nach einem Entnehmen eines medizinischen Instruments oder eines Trägers mit einer eindeutigen Kennzeichnung aus dem zweiten Innenraum - und ggf. nach Durchführung der Schritte gemäss Ausführungsbeispiel 19 - ein der eindeutigen Kennzeichnung entsprechender oder dieser zugeordneter Eintrag aus der Datenbank gelöscht wird; und/oder in der Datenbank vermerkt wird, dass und/oder wann das Instrument oder der Träger mit besagter eindeutigen Kennzeichnung entnommen wurde;
26. System zum Sterilisieren und zum sterilen Lagern und/oder Aufbewahren medizinischer Instrumente, welchem zu sterilisierende medizinische, insbesondere zahnmedizinische, Instrumente als Sterilisationsgut zugeführt und sterilisierte medizinische Instrumente als Sterilgut entnommen werden können, besagtes System umfassend:
   a. einen Sterilisator (1), insbesondere einen Autoklaven, umfassend:
      i. einen ersten Innnenraum (11) zur Aufnahme von Sterilisationsgut,
      ii. eine Entladeöffnung (12) zum Entnehmen von Sterilgut aus dem ersten Innenraum,
      iii. eine Entladeklappe (121), mittels welcher insbesondere die Entladeöffnung fluid- und druckdicht verschlossen werden kann;
   b. eine Sicherheitswerkbank (2), insbesondere eine Laminar Flow Box, umfassend:
      i. einen zweiten Innenraum (21), insbesondere zur Aufbewahrung von Sterilgut,
      ii. eine Befüllungsöffnung (23) zur Befüllung des zweiten Innenraums mit Sterilgut,
      iii. eine Entnahmeöffnung zur Entnahme von Sterilgut aus dem zweiten Innenraum;
   dadurch gekennzeichnet, dass
   c. die Entladeöffnung mit der Befüllungsöffnung verbunden ist, insbesondere in die Befüllungsöffnung mündet und/oder geführt ist, insbesondere in die Befüllungsöffnung hineinragt.
27. Verfahren zum Bereitstellen eines Systems zum Sterilisieren und zum sterilen Lagern und/oder Aufbewahren medizinischer Instrumente, umfassend die Schritte:
   a. Bereitstellen eines Sterilisators (1), insbesondere einen Autoklaven, umfassend:
      i. einen ersten Innnenraum (11) zur Aufnahme von Sterilisationsgut,
      ii. eine Entladeöffnung (12) zum Entnehmen von Sterilgut aus dem ersten Innenraum,
      iii. eine Entladeklappe (121), mittels welcher insbesondere die Entladeöffnung fluid- und druckdicht verschlossen werden kann;
   b. Bereitstellen einer Sicherheitswerkbank (2), insbesondere einer Laminar Flow Box, umfassend:
      i. einen zweiten Innenraum (21), insbesondere zur Aufbewahrung von Sterilgut,
      ii. eine Befüllungsöffnung (23) zur Befüllung des zweiten Innenraums mit Sterilgut,
      iii. eine Entnahmeöffnung zur Entnahme von Sterilgut aus dem zweiten Innenraum;
   gekennzeichnet durch
   c. Verbinden der Entladeöffnung mit der Befüllungsöffnung, insbesondere derart, dass die Entladeöffnung in die Befüllungsöffnung mündet und/oder geführt ist, insbesondere in die Befüllungsöffnung hineinragt.
28. System oder Verfahren nach Ausführungsbeispiel 26 oder 27, dadurch gekennzeichnet, dass der Sterilisator ferner aufweist:
   a. eine Beladeöffnung (13) zum Einbringen von Sterilisationsgut in den ersten Innenraum und
   b. eine Beladeklappe (131), mittels welcher die Beladeöffnung fluid- und druckdicht verschlossen werden kann, wobei
   c. die Beladeöffnung von der Entladeöffnung entfernt angeordnet ist, vorzugsweise an einer anderen, insbesondere gegenüberliegenden Seite des Sterilisators.
29. System oder Verfahren nach einem der vorangehenden Ausführungsbeispiele, dadurch gekennzeichnet, dass die Entladeöffnung fluiddicht mit der Befüllungsöffnung verbunden ist bzw. wird, und/oder eine fluiddichte Verbindung zwischen der Entladeöffnung und der Befüllungsöffnung vorgesehen ist bzw. wird.
30. System oder Verfahren nach einem der vorangehenden Ausführungsbeispiele, dadurch gekennzeichnet, dass die Entnahmeöffnung eine Arbeitsöffnung (22) ist, durch welche insbesondere
   a. Bedienpersonal in den zweiten Innenraum greifen kann, insbesondere um im zweiten Innenraum befindliches Sterilgut zu entnehmen oder innerhalb des zweiten Innenraums zu bewegen, insbesondere zu verschieben oder umzuplatzieren;
   b. durch den zweiten Innenraum und die Entladeöffnung in den ersten Innenraum greifen kann, insbesondere um Sterilgut aus dem ersten Innenraum zu entnehmen und durch die Entladeöffnung in den zweiten Innenraum zu verbringen; wobei
   c. die Arbeitsöffnung vorzugsweise verschliessbar ist, insbesondere mittels einer Abdeckung, welche insbesondere klappbar oder verschiebbar ausgeführt ist.
31. System oder Verfahren nach einem der vorangehenden Ausführungsbeispiele, dadurch gekennzeichnet, dass ein Verriegelungsmechanismus vorgesehen ist bzw. wird, welcher dazu eingerichtet ist, die Entladeklappe nach einem Verschliessen zu verriegeln, so dass diese erst wieder geöffnet werden kann, nachdem ein Sterilisationsprozess durchgeführt wurde.
32. System oder Verfahren nach Ausführungsbeispiel 31, dadurch gekennzeichnet, dass der Verriegelungsmechanismus dazu eingerichtet ist, ein Öffnen der Entladeklappe zu verhindern, solange die Beladeklappe geöffnet ist, und/oder ein Öffnen der Beladeklappe zu verhindern, solange die Entladeklappe geöffnet ist.
33. System oder Verfahren nach einem der vorangehenden Ausführungsbeispiele, dadurch gekennzeichnet, dass die Befüllungsöffnung und die Entnahmeöffnung an unterschiedlichen Seiten der Sicherheitswerkbank vorgesehen sind.
34. System oder Verfahren nach einem der vorangehenden Ausführungsbeispiele, dadurch gekennzeichnet, dass das System, insbesondere der Sterilisator, dazu eingerichtet ist, Daten oder Informationen über einen, insbesondere betreffend den letzten, Sterilisierungsprozess und/oder einen Status, insbesondere einen Wartungs- oder Fehlerstatus, des Sterilisators, an eine Datenverarbeitungsanlage zu übermitteln, insbesondere zur Speicherung in einer Datenbank.
35. System oder Verfahren nach einem der vorangehenden Ausführungsbeispiele, dadurch gekennzeichnet, dass im zweiten Innenraum eine Ablage, insbesondere ein Regal oder Ständer vorgesehen ist, welche insbesondere eine Vielzahl von Fächern enthält, die für die Aufnahme von Trägern geeignet sind, wobei insbesondere jedes Fach
   a. durch eine, insbesondere eindeutige, Fachnummer identifizierbar ist und/oder
   b. dazu ausgelegt ist, genau einen Träger und/oder genau eine Art von Trägern aufzunehmen.
36. System oder Verfahren nach einem der vorangehenden Ausführungsbeispiele, dadurch gekennzeichnet, dass zumindest an und/oder für einen Teil der Fächer
   a. ein Signalgeber, insbesondere ein optischer Signalgeber wie beispielsweise eine Leuchtdiode (LED), vorgesehen ist oder wird;
   b. eine (zweite) eindeutige Kennzeichnung vorgesehen ist oder wird, insbesondere in Form eines Strichcodes, eines QR-Codes, einer Zahl oder Zeichenfolge; und/oder
   c. jeweils ein RFID-Lesegerät vorgesehen ist oder wird, welches mit einer Datenverarbeitungsanlage verbunden und/oder verbindbar ist oder verbunden wird, insbesondere um eindeutige (erste) Kennzeichnungen auszulesen, welche auf einem oder mehreren RFID-Chips gespeichert sind, welche mit medizinischen Instrumenten und/oder einem Träger verbunden sind, die sich in dem entsprechenden Fach befinden oder in das entsprechende Fach verbracht werden.
37. System oder Verfahren nach einem der vorangehenden Ausführungsbeispiele, dadurch gekennzeichnet, dass das System ein Erfassungsgerät für Kennzeichnungen umfasst, welche insbesondere an medizinischen Instrumenten, Trägern oder Fächern vorgesehen sind, insbesondere einen Barcode-Scanner, einen QR-Code Scanner und/oder ein RFID-Lesegerät, wobei das Erfassungsgerät insbesondere mit einer Datenverarbeitungsanlage verbindbar oder verbunden ist und/oder insbesondere dazu eingerichtet ist, erfasste Kennzeichnungen an eine Datenverarbeitungsanlage zu übermitteln, insbesondere zur Speicherung in einer Datenbank.
38. System oder Verfahren nach einem der vorangehenden Ausführungsbeispiele, dadurch gekennzeichnet, dass eine Vielzahl von Trägern zur Aufnahme medizinischer Instrumente vorgesehen sind, von denen jeder eine eindeutige Kennzeichnung, insbesondere in Form eines Strichcodes, eines QR-Codes, einer Zahl oder einer Zeichenfolge, aufweist, wobei sich insbesondere die eindeutige Kennzeichnung eines jeden Trägers von der eindeutigen Kennzeichnung aller übrigen Träger unterscheidet.
39. System oder Verfahren nach einem der vorangehenden Ausführungsbeispiele, dadurch gekennzeichnet, dass eine Vielzahl medizinischen Instrumenten vorgesehen sind, von denen jeder eine eindeutige Kennzeichnung, insbesondere in Form eines Strichcodes, eines QR-Codes, einer Zahl oder einer Zeichenfolge, aufweist, wobei sich insbesondere die eindeutige Kennzeichnung eines jeden medizinischen Instrumenten von der eindeutigen Kennzeichnung aller übrigen medizinischen Instrumente unterscheidet, und insbesondere von der eindeutigen Kennzeichnung aller Träger unterscheidet.
40. System oder Verfahren nach einem der vorangehenden Ausführungsbeispiele, welches mit einer Datenverarbeitungsanlage verbunden oder verbindbar ist, welche dazu ausgelegt ist, eine Datenbank zu speichern und/oder zu bearbeiten, in welcher zu einer, insbesondere jeder, eindeutigen Kennung, welche an einem medizinischen Instrument oder an einem Träger vorgesehen ist, gespeichert werden kann:
   a. ein Zeitpunkt, insbesondere ein Datum, und/oder ein Zeitraum an bzw. in welchem (letztmalig) eine Sterilisierung des zugehörigen medizinischen Instruments und/oder im Träger befindlicher medizinsicher Instrumente im Sterilisator erfolgt ist;
   b. Prozessparameter, insbesondere deren Ist- und/oder Sollwerte, und/oder sonstige Informationen betreffend den Sterilisierungsprozess oder den Sterilisator;
   c. ein Verfalldatum, nach welchem das medizinische Instrument oder die im Träger befindlichen medizinischen Instrumente nicht ehr benutzt werden sollen oder dürfen;
   d. eine Position, insbesondere ein Fach, in der Ablage, an bzw. in welchem sich das medizinische Instrument und/oder der Träger befindet.

## Patentansprüche

1. Verfahren zum Sterilisieren von medizinischen Instrumenten, umfassend die Schritte
a. Einbringen von Sterilisationsgut umfassend zu sterilisierende medizinische Instrumente in einen ersten Innenraum (11) eines Sterilisators (1), insbesondere eines Autoklaven;
b. Sterilisieren des Sterilisationsguts im Sterilisator, insbesondere mittels eines Sterilisierungsprozesses des Sterilisators, um Sterilgut umfassend die medizinischen Instrumente zu erhalten;
c. Entnehmen des Sterilguts durch eine Entladeöffnung (12) des Sterilisators;
d. Einbringen des Sterilguts in einen zweiten Innenraum (21) einer Sicherheitswerkbank (2), insbesondere einer Laminar Flow Box durch eine Befüllungsöffnung (23) der Sicherheitswerkbank;
e. Lagern des Sterilguts im zweiten Innenraum (21) der Sicherheitswerkbank;
f. Entnehmen des Sterilguts durch eine Entnahmeöffnung der Sicherheitswerkbank; wobei
g. die Entladeöffnung mit der Befüllungsöffnung verbunden ist oder wird, insbesondere derart, dass die Entladeöffnung in die Befüllungsöffnung mündet und/oder geführt ist, insbesondere in die Befüllungsöffnung hineinragt; und
h. das Sterilgut dem zweiten Innenraum erst unmittelbar vor einem medizinischen, insbesondere zahnmedizinischen, Eingriff entnommen wird, bei welchem die medizinischen Instrumente eingesetzt werden, **dadurch gekennzeichnet, dass**
i. das Sterilisationsgut auf oder in einem oder mehreren Trägern, insbesondere in Form von (wieder-)verschliessbaren Boxen, in den ersten Innenraum eingebracht wird, wobei insbesondere eine Vielzahl von unterschiedlichen Trägern und/oder Trägern unterschiedlicher Art vorhanden sind oder verwendet werden, welche sich insbesondere dadurch unterscheiden, dass sie dazu ausgelegt sind, ein unterschiedliches Sortiment an medizinischen Instrumenten aufzunehmen, wobei insbesondere eine erste Art von Träger dazu ausgelegt ist, ein erstes Sortiment aufzunehmen, und eine zweite Art von Träger dazu ausgelegt ist, ein zweites Sortiment aufzunehmen, welches sich von dem ersten Sortiment unterscheidet.

2. Verfahren nach dem Anspruch 1, **dadurch gekennzeichnet, dass**
a. das Sterilisationsgut auf oder in dem einen oder mehreren Trägern sterilisiert wird,
b. auf oder in dem einen oder mehreren Trägern dem Sterilisator als Sterilgut entnommen wird,
c. das Sterilgut auf oder in dem einem oder mehreren Trägern im zweiten Innenraum (21) der Sicherheitswerkbank gelagert wird; und/oder
d. das Sterilgut auf oder in dem einem oder mehreren Trägern dem zweiten Innenraum entnommen wird.

3. Verfahren nach einem der vorangehenden Ansprüche, **dadurch gekennzeichnet, dass** zumindest ein Teil der medizinischen Instrumente und/oder ein Teil der Träger eine eindeutige Kennzeichnung aufweist, welche es insbesondere erlaubt, jedes Instrument und jeden Träger von jedem anderen Instrument bzw. jedem anderen Träger zu unterscheiden, wobei das Verfahren insbesondere den Schritt eines Anbringens der eindeutigen Kennzeichnung an einem oder mehreren Instrumenten oder Trägern umfasst.

4. Verfahren nach einem der vorangehenden Ansprüche, **dadurch gekennzeichnet, dass** zumindest für einen Teil der medizinischen Instrumente mit einer eindeutigen Kennzeichnung und/oder einen Teil der Träger mit einer eindeutigen Kennzeichnung ein Zeitpunkt, insbesondere ein Datum, und/oder ein Zeitraum an bzw. in welchem letztmalig eine Sterilisation im Sterilisator erfolgt ist, in einer Datenbank der entsprechenden eindeutigen Kennzeichnung zugeordnet und/oder gespeichert wird, gegebenenfalls zusammen mit weiteren Daten betreffend einen entsprechenden Sterilisierungsprozess, insbesondere den Sterilisierungsprozess gemäss Merkmal 1.b) von Anspruch 1.

5. Verfahren nach dem vorangehenden Anspruch, dass zumindest für den Teil der medizinischen Instrumente mit eindeutiger Kennzeichnung und/oder den Teil der Träger mit eindeutiger Kennzeichnung Informationen betreffend die Positionen gespeichert werden, an welchen die Instrumente oder Träger im zweiten Innenraum gelagert werden; insbesondere für mindestens ein Instrument oder für mindestens einen Träger gespeichert wird, in welchem Fach eines im zweiten Innenraum vorgesehenen Regals oder Gestells die Lagerung erfolgt.

6. Verfahren nach einem der vorangehenden Ansprüche, **dadurch gekennzeichnet, dass** vor, während oder unmittelbar nach einem Eingriff die eindeutige Kennzeichnung eines verwendeten oder zur Verwendung vorgesehenen Instruments oder mit Instrumenten bestückten Trägers und/oder die für das zugehörige Instrument oder den zugehörigen Träger in der Datenbank gespeicherten Daten oder Informationen, insbesondere betreffend den letzten Sterilisierungsprozess, zumindest teilweise in eine Patientenakte oder ein Patientendossier übertragen oder kopiert werden, welche(s) dem Patienten zugeordnet ist, an welchem der Eingriff durchgeführt werden soll, wird oder wurde, insbesondere in eine elektronische Patientenakte oder ein elektronisches Patientendossier.

7. Verfahren nach einem der vorangehenden Ansprüche, **dadurch gekennzeichnet, dass** vor einem Eingriff, insbesondere vor einem geplanten Eingriff, anhand der eindeutigen Kennzeichnung eines für eine Verwendung vorgesehenen Instruments oder mit Instrumenten bestückten Trägers anhand von in der Datenbank gespeicherten Daten und Informationen überprüft wird, dass ein Verfalldatum für dieses Instrument und/oder diesen Träger, insbesondere die Instrumente, mit welchen dieser Träger bestückt ist, noch nicht überschritten wurde und/oder in der Zukunft liegt.

8. Verfahren nach einem der vorangehenden Ansprüche, **dadurch gekennzeichnet, dass** vor, während oder unmittelbar nach der Entnahme eines ersten Instruments oder mit Instrumenten bestückten ersten Trägers anhand von in der Datenbank gespeicherten Daten und Informationen ermittelt wird, ob ein gleichartiges zweites Instrument bzw. ein mit gleichen Instrumenten bestückter zweiter Träger existiert, insbesondere im zweiten Innenraum der Sicherheitswerkbank gelagert wird, für welches bzw. für welchen ein Verfalldatum weniger weit in der Zukunft liegt als ein Verfalldatum für das erste Instrument bzw. für den ersten Träger, insbesondere mindestens mehrere Tage weniger weit in der Zukunft.

9. Verfahren nach dem vorangehenden Anspruch, **dadurch gekennzeichnet, dass** die in diesem Anspruch beschriebenen Schritte so oft bzw. so lange wiederholt werden, bis kein Instrument oder Träger mehr ermittelt wird, für welches bzw. für welchen das Verfalldatum weniger weit in der Zukunft liegt als für das zuletzt ermittelte Instrument bzw. den zuletzt ermittelten Träger.

10. Verfahren nach einem der vorangehenden Ansprüche, **dadurch gekennzeichnet, dass**
a. vor einem geplanten Eingriff und/oder während eines Eingriffs bestimmt wird, welche Art von Instrument und/oder welche Art von mit Instrumenten bestücktem Träger benötigt wird,
b. anhand von in der Datenbank gespeicherten Daten und Informationen ein Instrument und/oder ein Träger der bestimmten Art ermittelt wird, für welchen ein Verfalldatum am wenigsten weit in der Zukunft liegt;
c. das ermittelte Instrument oder der ermittelte Träger dem zweiten Innenraum entnommen wird, um den Eingriff durchzuführen.

11. Verfahren nach dem vorangehenden Anspruch, wobei das ermittelte Instrument oder der (zuletzt) ermittelte Träger anhand er eindeutigen Kennzeichnung, die es oder er aufweist, identifiziert oder lokalisiert wird.

12. Verfahren nach einem der vorangehenden Ansprüche, **dadurch gekennzeichnet, dass** nach einem Entnehmen eines medizinischen Instruments oder eines Trägers mit einer eindeutigen Kennzeichnung aus dem zweiten Innenraum - und ggf. nach Durchführung der Schritte gemäss Anspruch 19 - ein der eindeutigen Kennzeichnung entsprechender oder dieser zugeordneter Eintrag aus der Datenbank gelöscht wird; und/oder in der Datenbank vermerkt wird, dass und/oder wann das Instrument oder der Träger mit besagter eindeutigen Kennzeichnung entnommen wurde;

13. System zum Sterilisieren und zum sterilen Lagern und/oder Aufbewahren medizinischer Instrumente, welchem zu sterilisierende medizinische, insbesondere zahnmedizinische, Instrumente als Sterilisationsgut zugeführt und sterilisierte medizinische Instrumente als Sterilgut entnommen werden können, besagtes System umfassend:
a. einen Sterilisator (1), insbesondere einen Autoklaven, umfassend:
i. einen ersten Innnenraum (11) zur Aufnahme von Sterilisationsgut,
ii. eine Entladeöffnung (12) zum Entnehmen von Sterilgut aus dem ersten Innenraum,
iii. eine Entladeklappe (121), mittels welcher insbesondere die Entladeöffnung fluid- und druckdicht verschlossen werden kann;
b. eine Sicherheitswerkbank (2), insbesondere eine Laminar Flow Box, umfassend:
i. einen zweiten Innenraum (21), insbesondere zur Aufbewahrung von Sterilgut,
ii. eine Befüllungsöffnung (23) zur Befüllung des zweiten Innenraums mit Sterilgut,
iii. eine Entnahmeöffnung zur Entnahme von Sterilgut aus dem zweiten Innenraum;
**dadurch gekennzeichnet, dass**
c. die Entladeöffnung mit der Befüllungsöffnung verbunden ist, insbesondere in die Befüllungsöffnung mündet und/oder geführt ist, insbesondere in die Befüllungsöffnung hineinragt.

14. Verfahren zum Bereitstellen eines Systems zum Sterilisieren und zum sterilen Lagern und/oder Aufbewahren medizinischer Instrumente, umfassend die Schritte:
a. Bereitstellen eines Sterilisators (1), insbesondere einen Autoklaven, umfassend:
i. einen ersten Innnenraum (11) zur Aufnahme von Sterilisationsgut,
ii. eine Entladeöffnung (12) zum Entnehmen von Sterilgut aus dem ersten Innenraum,
iii. eine Entladeklappe (121), mittels welcher insbesondere die Entladeöffnung fluid- und druckdicht verschlossen werden kann;
b. Bereitstellen einer Sicherheitswerkbank (2), insbesondere einer Laminar Flow Box, umfassend:
i. einen zweiten Innenraum (21), insbesondere zur Aufbewahrung von Sterilgut,
ii. eine Befüllungsöffnung (23) zur Befüllung des zweiten Innenraums mit Sterilgut,
iii. eine Entnahmeöffnung zur Entnahme von Sterilgut aus dem zweiten Innenraum;
**gekennzeichnet durch**
c. Verbinden der Entladeöffnung mit der Befüllungsöffnung, insbesondere derart, dass die Entladeöffnung in die Befüllungsöffnung mündet und/oder geführt ist, insbesondere in die Befüllungsöffnung hineinragt.

15. System oder Verfahren nach Anspruch 13 oder 14, **dadurch gekennzeichnet, dass** das System, insbesondere der Sterilisator, dazu eingerichtet ist, Daten oder Informationen über einen, insbesondere betreffend den letzten, Sterilisierungsprozess und/oder einen Status, insbesondere einen Wartungs- oder Fehlerstatus, des Sterilisators, an eine Datenverarbeitungsanlage zu übermitteln, insbesondere zur Speicherung in einer Datenbank.

16. System oder Verfahren nach einem der Ansprüche 13 bis 15, **dadurch gekennzeichnet, dass** im zweiten Innenraum eine Ablage, insbesondere ein Regal oder Ständer vorgesehen ist, welche insbesondere eine Vielzahl von Fächern enthält, die für die Aufnahme von Trägern geeignet sind, wobei insbesondere jedes Fach
a. durch eine, insbesondere eindeutige, Fachnummer identifizierbar ist und/oder
b. dazu ausgelegt ist, genau einen Träger und/oder genau eine Art von Trägern aufzunehmen.

17. System oder Verfahren nach einem der Ansprüche 13 bis 16, **dadurch gekennzeichnet, dass** zumindest an und/oder für einen Teil der Fächer
a. ein Signalgeber, insbesondere ein optischer Signalgeber wie beispielsweise eine Leuchtdiode (LED), vorgesehen ist oder wird;
b. eine (zweite) eindeutige Kennzeichnung vorgesehen ist oder wird, insbesondere in Form eines Strichcodes, eines QR-Codes, einer Zahl oder Zeichenfolge; und/oder
c. jeweils ein RFID-Lesegerät vorgesehen ist oder wird, welches mit einer Datenverarbeitungsanlage verbunden und/oder verbindbar ist oder verbunden wird, insbesondere um eindeutige (erste) Kennzeichnungen auszulesen, welche auf einem oder mehreren RFID-Chips gespeichert sind, welche mit medizinischen Instrumenten und/oder einem Träger verbunden sind, die sich in dem entsprechenden Fach befinden oder in das entsprechende Fach verbracht werden.

18. System oder Verfahren nach einem der Ansprüche 13 bis 17, **dadurch gekennzeichnet, dass** das System ein Erfassungsgerät für Kennzeichnungen umfasst, welche insbesondere an medizinischen Instrumenten, Trägern oder Fächern vorgesehen sind, insbesondere einen Barcode-Scanner, einen QR-Code Scanner und/oder ein RFID-Lesegerät, wobei das Erfassungsgerät insbesondere mit einer Datenverarbeitungsanlage verbindbar oder verbunden ist und/oder insbesondere dazu eingerichtet ist, erfasste Kennzeichnungen an eine Datenverarbeitungsanlage zu übermitteln, insbesondere zur Speicherung in einer Datenbank.

19. System oder Verfahren nach einem der Ansprüche 13 bis 18, **dadurch gekennzeichnet, dass** eine Vielzahl von Trägern zur Aufnahme medizinischer Instrumente vorgesehen sind, von denen jeder eine eindeutige Kennzeichnung, insbesondere in Form eines Strichcodes, eines QR-Codes, einer Zahl oder einer Zeichenfolge, aufweist, wobei sich insbesondere die eindeutige Kennzeichnung eines jeden Trägers von der eindeutigen Kennzeichnung aller übrigen Träger unterscheidet.

20. System oder Verfahren nach einem der Ansprüche 13 bis 19, **dadurch gekennzeichnet, dass** eine Vielzahl medizinischen Instrumenten vorgesehen sind, von denen jeder eine eindeutige Kennzeichnung, insbesondere in Form eines Strichcodes, eines QR-Codes, einer Zahl oder einer Zeichenfolge, aufweist, wobei sich insbesondere die eindeutige Kennzeichnung eines jeden medizinischen Instrumenten von der eindeutigen Kennzeichnung aller übrigen medizinischen Instrumente unterscheidet, und insbesondere von der eindeutigen Kennzeichnung aller Träger unterscheidet.

21. System oder Verfahren nach einem der Ansprüche 13 bis 20, welches mit einer Datenverarbeitungsanlage verbunden oder verbindbar ist, welche dazu ausgelegt ist, eine Datenbank zu speichern und/oder zu bearbeiten, in welcher zu einer, insbesondere jeder, eindeutigen Kennung, welche an einem medizinischen Instrument oder an einem Träger vorgesehen ist, gespeichert werden kann:
a. ein Zeitpunkt, insbesondere ein Datum, und/oder ein Zeitraum an bzw. in welchem (letztmalig) eine Sterilisierung des zugehörigen medizinischen Instruments und/oder im Träger befindlicher medizinsicher Instrumente im Sterilisator erfolgt ist;
b. Prozessparameter, insbesondere deren Ist- und/oder Sollwerte, und/oder sonstige Informationen betreffend den Sterilisierungsprozess oder den Sterilisator;
c. ein Verfalldatum, nach welchem das medizinischen Instrument oder die im Träger befindlichen medizinischen Instrumente nicht ehr benutzt werden sollen oder dürfen;
d. eine Position, insbesondere ein Fach, in der Ablage, an bzw. in welchem sich das medizinische Instrument und/oder der Träger befindet.
